(19) Europäisches Patentamt
European Patent Office
Office européen des brevets

(11) **EP 1 776 920 A2**

(12) **EUROPEAN PATENT APPLICATION**

(43) Date of publication:
**25.04.2007 Bulletin 2007/17**

(51) Int Cl.:
***A61B 5/021*** *(2006.01)*

(21) Application number: **06021662.9**

(22) Date of filing: **16.10.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **18.10.2005 JP 2005303272**

(71) Applicant: **Omron Healthcare Co., Ltd.
Kyoto 615-0084 (JP)**

(72) Inventors:
• **Sawanoi, Yukiya
Omron Healthcare Co., Ltd.
Kyoto-shi
Kyoto 615-0084 (JP)**

• **Eda, Kenji
Omron Healthcare Co., Ltd.
Kyoto-shi
Kyoto 615-0084 (JP)**
• **Tanaka, Takahide
Omron Healthcare Co., Ltd.
Kyoto-shi
Kyoto 615-0084 (JP)**
• **Kishimoto, Hiroshi
Omron Healthcare Co., Ltd.
Kyoto-shi
Kyoto 615-0084 (JP)**

(74) Representative: **Kilian, Helmut
Wilhelms, Kilian & Partner
Patentanwälte
Eduard-Schmid-Strasse 2
81541 München (DE)**

(54) **Electronic blood pressure monitor capable of storing measurement data**

(57) In an electronic blood pressure monitor, an input of measurement condition specifying information for specifying a measurement condition at blood pressure measurement among a plurality of measurement conditions, such as an input of time information, is received (step S4A). Based on the received measurement condition specifying information, at least one measurement condition of a patient at blood pressure measurement is determined (step S6A). A calculated blood pressure value is recorded by being associated with the measurement condition determined as such (step S 18).

FIG.4

EP 1 776 920 A2

**Description**

BACKGROUND OF THE INVENTION

Field of the Invention

The present invention relates to an electronic blood pressure monitor, and particularly to an electronic blood pressure monitor that can be used at home.

Description of the Background Art

In recent years, as self-medication has become prevalent, a blood pressure monitor capable of measuring blood pressure at home has rapidly achieved widespread use. Such a home blood pressure monitor has a function of informing a user by a buzzer or a display device when a measurement value exceeds a certain value, and a function of recording and displaying blood pressure values measured at different times.
Blood pressure is one of the indicators for analyzing a circulatory disease. Risk analysis based on blood pressure is effective for preventing cardiovascular system diseases such as cerebral strokes, cardiac failure, and cardiac infarction. Particularly, morning hypertension, which causes blood pressure rise in the early morning, relates to a cardiac disease, cerebral strokes, and the like. Furthermore, a symptom in the morning hypertension that causes blood pressure surge approximately one hour to one and a half hour after waking up, which symptom is referred to as morning surge, is proven to have a causal relationship with cerebral strokes. This is described in "On Morning Hypertension and Risk for Cerebrovascular Accident", Kazuomi Kario, Journal of Blood . Pressure, November Issue, sentan igaku-sha Ltd., November 1, 2002, vol. 9, no. 11, p. 94-47. Accordingly, it can be said that the grasping of the correlation between time (lifestyle habit) and changes in blood pressure is useful for risk analysis of cardiovascular system diseases.
Thus, Japanese Patent Laying-Open No. 2004-261452 proposes a blood pressure monitor capable of risk analysis of cardiovascular system diseases based on the correlation between time (lifestyle habit) and changes in blood pressure. According to this document, a measured blood pressure value is associated with time point information and condition information at measurement and stored. It is disclosed that an average value of the blood pressure values measured at each of particular time slots such as, for example, a morning time slot and an evening time slot is calculated, so that a risk value is calculated based on the calculation result and displayed.
In Japanese Patent Laying-Open No. 2004-261452, however, the time point information associated with the blood pressure values and stored is a time point obtained from a clock function incorporated in the blood pressure monitor, and the measured blood pressure values are automatically sorted based on the time point and stored. Accordingly, a condition such as after waking up or before sleeping, which condition is applied to the measurement values, may become meaningless particularly for a subject (patient) such as a shift worker whose living cycle is different from that of ordinary people.

SUMMARY OF THE INVENTION

The present invention is made to overcome the problem described above. An object of the present invention is to provide an electronic blood pressure monitor capable of determining a measurement condition of a patient at blood pressure measurement. Another object is to thereby provide capability of calculating an evaluated amount (risk value) having high reliability.
In order to achieve the above-described objects, an electronic blood pressure monitor according to an aspect of the present invention includes: a cuff attachable to a blood pressure measurement site; a pressurizing and depressurizing unit for adjusting pressure applied to the cuff; a pressure detecting unit for detecting pressure in the cuff; a blood pressure calculating unit for calculating blood pressure from a signal obtained at the pressure detecting unit; a storing unit for storing data of the calculated blood pressure; and a determining unit for, based on measurement condition specifying information for specifying a measurement condition at blood pressure measurement, determining at least one corresponding measurement condition among a plurality of types of blood pressure measurement conditions at blood pressure measurement.
Here, the "measurement condition" refers to a condition indicating a body state of a patient at blood pressure measurement, and refers to, for example, after waking up, before sleeping, before exercising, after exercising, before meal, after meal, before taking medicine, after taking medicine, a normal state, and others.
Preferably, the electronic blood pressure monitor further includes a timer unit for recording a time point, and a receiving unit for receiving from a subject (patient) an input of time information as the measurement condition specifying information. The received time information and information on the measurement condition are associated and further stored in the storing unit. The determining unit determines the measurement condition at blood pressure measurement, based on time point data output from the timer unit and the time information stored in the storing unit. At each blood pressure measurement, the measurement condition determined by the determining unit is related to the data of the blood pressure calculated by the blood pressure calculating unit, and stored in the storing unit.

Preferably, the determining unit retrieves the information on the measurement condition stored in the storing unit, based on the time point data and the time information, and thereby determines the measurement condition.

Preferably, in the storing unit, a storage region is provided for each of the measurement conditions. The data of the blood pressure is stored in the storage region corresponding to the information on the measurement condition.

Preferably, the plurality of types of measurement conditions include a measurement condition indicating any of blood pressure measurement after waking up and blood pressure measurement before sleeping.

Preferably, the electronic blood pressure monitor further includes a timer unit for recording a time point, and a receiving unit for receiving from a subject (patient) an input of time information as the measurement condition specifying information. At each blood pressure measurement, the electric blood pressure monitor relates time point data output from the timer unit to the data of the blood pressure calculated by the blood pressure calculating unit, for storage in the storing unit. The determining unit determines the measurement condition at blood pressure measurement based on the received time information and the time point data related to the data of the blood pressure in the storing unit.

Preferably, the plurality of types of measurement conditions correspond to a "period", the period including time, a day of a week, a day, the week, a month, a season, and a year. The time information is information on the period.

Preferably, the plurality of types of measurement conditions correspond to a "time period". The time information is information on commencement of the time period and information on termination of the time period.

Furthermore, the plurality of types of measurement conditions may correspond to a "time period". The time information may be information on any of commencement and termination of the time period, and information on a time period duration.

Furthermore, the plurality of types of measurement conditions may correspond to a "time period". A time period duration of the time period may be predetermined. The time information may be information on any of commencement and termination of the time period.

Preferably, the electronic blood pressure monitor further includes a receiving unit for receiving an input of voice information as the measurement condition specifying information. The determining unit performs a process of recognizing the received voice information, retrieves information on the measurement condition corresponding to the recognized voice information, and thereby determines the measurement condition at the blood pressure measurement. At each blood pressure measurement, the electronic blood pressure monitor relates the measurement condition determined by the determining unit to the data of the blood pressure calculated by the blood pressure calculating unit, for storage in the storing unit.

Preferably, the electronic blood pressure monitor further includes a receiving unit for additionally serving as at least one manipulation unit manipulated for receiving an external instruction, and for receiving, as the measurement condition specifying information, an input of at least one piece of manipulation information from information on a manipulation sequence of the manipulation unit, information on a number of manipulations of the manipulation unit, and information on a manipulation interval of the manipulation unit. The determining unit retrieves information on the measurement condition associated in advance with the received manipulation information, and thereby determines the measurement condition at blood pressure measurement. At each pressure measurement, the measurement condition determined by the determining unit is related to the data of the blood pressure calculated by the blood pressure calculating unit, and stored in the storing unit.

Preferably, the electronic blood pressure monitor further includes a detecting unit for detecting any of a light quantity and a pressure amount, and a receiving unit for receiving, from the detecting unit, an input of a detected amount as the measurement condition specifying information. The determining unit compares the received detected amount and a predetermined threshold value, and determines the measurement condition at blood pressure measurement. At each blood pressure measurement, the measurement condition determined by the determining unit is related to the data of the blood pressure calculated by the blood pressure calculating unit, and stored in the storing unit.

Preferably, an input of the measurement condition specifying information is received from the receiving unit before blood pressure measurement. The "before blood pressure measurement" refers to time at least before calculation of blood pressure.

Preferably, an input of the measurement condition specifying information is received from the receiving unit after blood pressure measurement. The "after blood pressure measurement" refers to time at least after calculation of blood pressure.

Preferably, the electronic blood pressure monitor further includes a selecting unit for making a selection as to whether or not inputting is performed after blood pressure measurement. If the selecting unit selects to perform inputting, an input of the measurement condition specifying information is received from the receiving unit.

Preferably, the electronic blood pressure monitor further includes an evaluated amount calculating unit for calculating an evaluated amount based on the correlation between a first blood pressure data group including at least one item of blood pressure data corresponding to the single measurement condition, and a second blood pressure data group including at least one item of blood pressure data corresponding to a measurement condition different from the single measurement condition, in the blood pressure data stored in the storing unit.

Preferably, the electronic blood pressure monitor further includes an evaluated amount calculating unit for calculating an evaluated amount for a blood pressure data group including at least one item of blood pressure data corresponding to the single measurement condition in the blood pressure data stored in the storing unit.

Preferably, the electronic blood pressure monitor further includes a display unit for displaying a result of blood pressure calculation performed by the blood pressure calculating unit and/or a result of evaluated amount calculation performed by the evaluated amount calculating unit.

According to the present invention, it is possible to determine the measurement condition of the subject (patient) at blood pressure measurement. It is thereby possible to apply an appropriate condition to the data on the measured blood pressure.

The foregoing and other objects, features, aspects and advantages of the present invention will become more apparent from the following detailed description of the present invention when taken in conjunction with the accompanying drawings.

BRIEF DESCRIPTION OF THE DRAWINGS

Fig. 1 is a schematic diagram of an electronic blood pressure monitor according to a first embodiment and its first to fifth modifications of the present invention.

Fig. 2 is an internal configuration diagram of the electronic blood pressure monitor according to the first embodiment and its first to fifth modifications of the present invention.

Figs. 3A and 3B are diagrams each showing an example of stored contents of a measurement result in a memory in the first embodiment and its first to fifth modifications of the present invention.

Fig. 4 is a flowchart of a main routine executed by a CPU in the electronic blood pressure monitor in the first embodiment of the present invention.

Figs. 5A-5C are diagrams each showing an example of a screen display when measurement time information is input as determination criterion information.

Figs. 6A and 6B are diagrams each showing an example of contents of a time correlation table.

Fig. 7 is a flowchart of a main routine executed by the CPU in the electronic blood pressure monitor in the first modification of the first embodiment of the present invention.

Fig. 8 is a flowchart of a main routine executed by the CPU in the electronic blood pressure monitor in the second modification of the first embodiment of the present invention.

Fig. 9 is a flowchart of a main routine executed by the CPU in the electronic blood pressure monitor in the third modification of the first embodiment of the present invention.

Fig. 10 is a flowchart of a main routine executed by the CPU in the electronic blood pressure monitor in the fourth modification of the first embodiment of the present invention.

Fig. 11 is a flowchart showing an interrupt process in the fourth modification of the first embodiment.

Fig. 12 is a diagram showing an example of stored contents of a measurement result in a memory in the fourth modification of the first embodiment.

Fig. 13 is a flowchart of a main routine executed by the CPU in the electronic blood pressure monitor in the fifth modification of the first embodiment of the present invention.

Fig. 14 is a diagram showing an example of a screen display when a specific state is input as a measurement result.

Fig. 15 is a schematic diagram of an electronic blood pressure monitor according to a second embodiment of the present invention.

Fig. 16 is an internal configuration diagram of the electronic blood pressure monitor according to the second embodiment of the present invention.

Fig. 17 is a diagram showing a correlation table of character data and measurement conditions.

Fig. 18 is a first diagram showing an example of a system configuration according to a third embodiment.

Fig. 19 is a second diagram showing an example of the system configuration according to the third embodiment.

DESCRIPTION OF THE PREFERRED EMBODIMENTS

The embodiments of the present invention will hereinafter be described in detail with reference to the drawings. The same characters are attached to the same or corresponding portions in the drawings, and the description thereof will not be repeated.

[First Embodiment]

(Configuration)

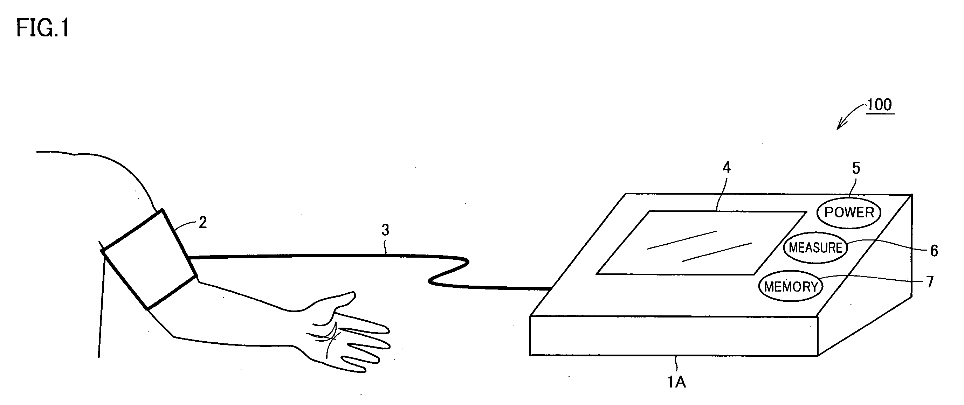

Fig. 1 is a schematic diagram of an electronic blood pressure monitor 100 according to a first embodiment of the present invention. Referring to Fig. 1, electronic blood pressure monitor 100 according to the present embodiment is provided with a blood pressure monitor main body 1A, a cuff 2 attached to a blood pressure measurement site of a subject (patient) for pneumatic pressurization, and an air pipe 3 connecting blood pressure monitor main body 1A to cuff 2.

Blood pressure monitor main body 1A has a display unit 4 allowing the patient to confirm the contents of display, and a power switch 5, a measurement switch 6, and a memory switch 7 that are provided to allow the patient to manipulate the blood pressure monitor externally.

Power switch 5 is manipulated to turn on/off the power supply of blood pressure monitor main body 1A. Measurement switch 6 is manipulated to instruct the start of blood pressure measurement. Memory switch 7 is manipulated to invoke stored blood pressure data.

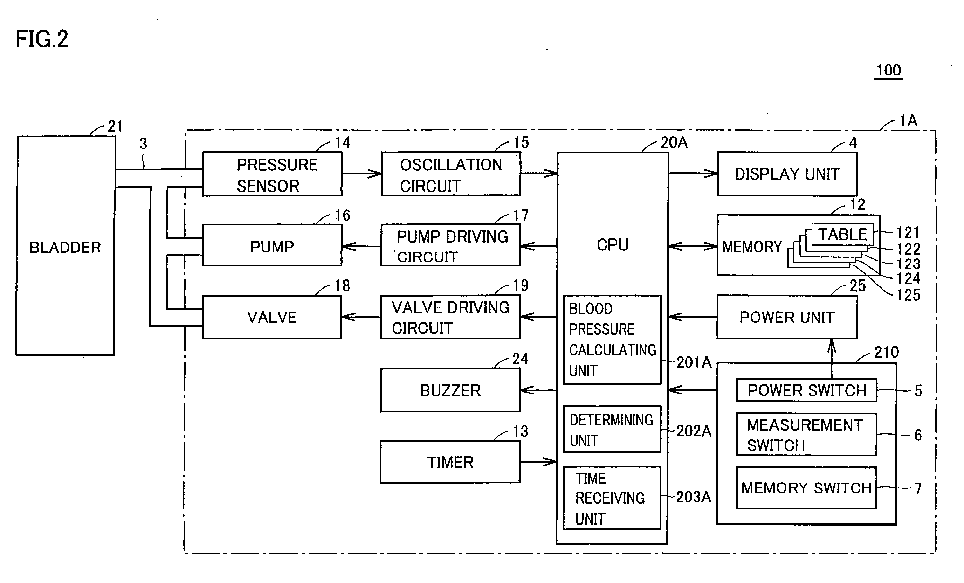

Fig. 2 shows an internal configuration of blood pressure monitor main body 1A. Referring to Fig. 2, blood pressure monitor main body 1A includes a pressure sensor 14, the capacitance of which varies depending on pressure in a bladder 21 embedded in cuff 2 (hereinafter referred to as "cuff pressure"), an oscillation circuit 15 outputting to a Central Processing Unit (CPU) 20A a signal having a oscillation frequency corresponding to a capacitance value of pressure sensor 14, a pump 16 and a valve 18 which are provided for adjusting a level of the cuff pressure, a pump driving circuit 17 driving pump 16, a valve driving circuit 19 for adjusting opening and closing positions of valve 18, and CPU 20A for intensively controlling and monitoring each unit. Furthermore, blood pressure monitor main body 1A includes display unit 4, a memory 12 where various types of data and programs are stored, a manipulation unit 210, a timer 13 performing a timer operation to output timing data, a buzzer 24, and a power unit 25 for supplying electric power. Bladder 21 is connected to pressure sensor 14, pump 16, and valve 18 via air pipe 3. CPU 20A converts the signal obtained from oscillation circuit 15 into a pressure signal and senses pressure. CPU 20A has a blood pressure calculating unit 201A, a determining unit 202A, and a time receiving unit 203A.

Manipulation unit 210 includes power switch 5, measurement switch 6, and memory switch 7 shown in Fig. 1.

When blood pressure is measured in the above-described configuration, CPU 20A uses blood pressure calculating unit 201A and applies a prescribed algorithm to the pressure data sensed based on the signal from oscillation circuit 15, so as to calculate blood pressure values, namely, systolic blood pressure and diastolic blood pressure, as well as a pulse rate. For such a measurement procedure, a well-known procedure conventionally provided can be applied, and hence the detailed description thereof will not be provided here.

In the present embodiment, CPU 20A uses determining unit 202A to determine a measurement condition at blood pressure measurement based on measurement condition specifying information. Furthermore, CPU 20A desirably has a function of calculating a blood pressure-related, evaluated amount for each of the determined measurement conditions, or a blood pressure-related, evaluated amount corresponding to more than one prescribed measurement conditions. A method of calculating an evaluated amount will be described later.

The "measurement condition specifying information" refers to information for specifying a measurement condition at blood pressure measurement, and includes an attribute at blood pressure measurement and determination criterion information. The "attribute" refers to information obtained as blood pressure is measured, such as, for example, time point data to be recorded, externally-input voice data, or a detected amount from a sensor or the like. The "determination criterion information" refers to information serving as a criterion for determining a measurement condition at blood pressure measurement, namely, information for defining the correlation between the attribute and the measurement condition. The determination criterion information may be input by the patient at blood pressure measurement, or predetermined before shipment as non-rewritable data in memory 12, for example, or subsequently set by the patient and stored.

In the first embodiment, the determination criterion information is, for example, time information such as a "period" or a "time period" for specifying the measurement condition. The "period" here refers to time, a day of the week, a day, a week, a month, a season, a year and the like. The "time period" refers to a span from a certain period to a certain period, such as a time slot (e.g. 7-9 o'clock).

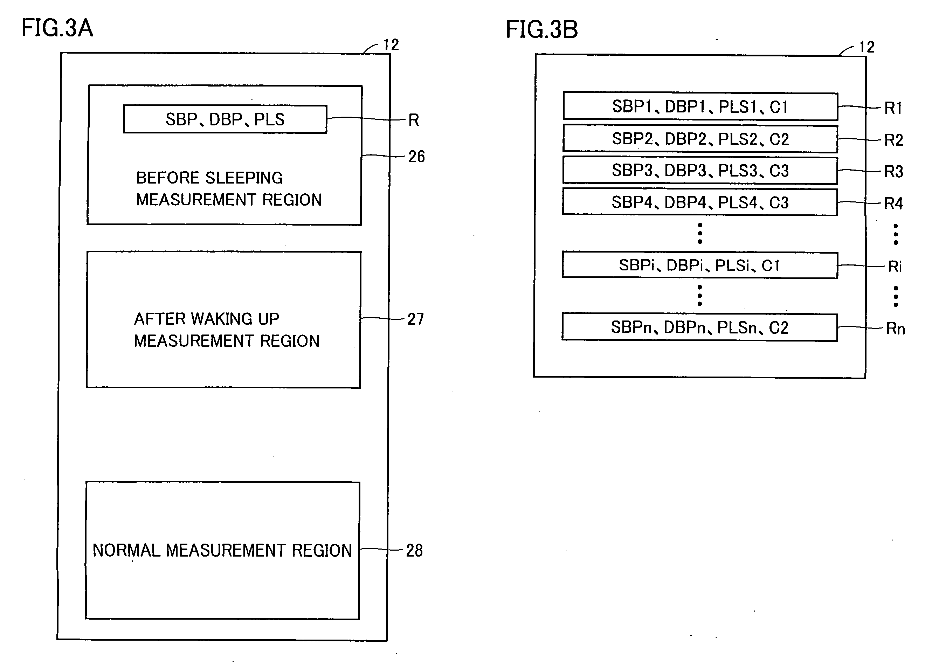

In the first embodiment, there are three measurement conditions including a time slot after waking up, a time slot before sleeping; and a normal time slot. An input of time information for specifying these measurement conditions (hereinafter referred to as "measurement time information") is received as the determination criterion information. A concrete example of contents of memory 12 in this case is shown in each of Figs. 3A and 3B.

Referring to Fig. 3A, memory 12 is provided in advance with storage regions 26, 27 and 28 intended for the measurement conditions, namely, the time slot before sleeping, the time slot after waking up, and the normal time slot, respectively. Measurement results are respectively stored in regions 26, 27 and 28 in the unit record R. Record R includes a systolic blood pressure data SBP indicating systolic blood pressure, a diastolic blood pressure data DBP indicating diastolic blood pressure, and pulse rate data PLS indicating a pulse rate. These items of data may be associated with the measurement conditions and stored in the regions, respectively, at each measurement, and the storage scheme is not limited to the one using record R. In addition, data of measurement time point (time point when measurement is started or completed) may further be stored in record R.

Referring to Fig. 3B, a measurement result and measurement condition information are paired and stored in memory 12. In Fig. 3B, a record Ri (i = 1, 2, 3, ..., n) in which a blood pressure value is associated with a measurement condition information is stored at each blood pressure measurement. In record Ri, systolic blood pressure data SBPi, diastolic blood pressure data DBPi, pulse rate data PLSi, and any of measurement condition data C1, C2 and C3 are stored. Measurement condition data C1, C2 and C3 correspond to the measurement conditions, namely, the time slot before sleeping, the time slot after waking up, and the normal time slot, respectively. In addition, data on a measurement time point (time point when measurement is started or completed) may further be stored in record Ri.

In the following description, items of blood pressure data are grouped per measurement condition, and stored in each of the memory regions, as shown in Fig. 3 A.

(Calculation of Evaluated Amount)

In the first embodiment of the present invention, an evaluated amount calculating unit in CPU 20A calculates, for example, a cardiovascular risk value as the above-described evaluated amount. The cardiovascular risk value is thought to be useful for preventing cardiovascular accidents such as cerebral strokes, cardiac failure, cerebral infarction, cerebral hemorrhage, subarachnoid hemorrhage, transient cerebral ischemic attack, fall, syncope, dizziness, stagger, cardiac infarction, angina pectoris, asymptomatic cardiac ischemia, arrhythmia, sudden death, dissecting aortic aneurysm, and ruptured aortic aneurysm. The evaluated amount calculated is displayed on display unit 4. A function of the evaluated amount calculating unit is implemented by a program corresponding thereto being read from memory 12 and executed by CPU 20A.

A method of calculating such an evaluated amount will be described.

■ First Example of Calculation

In a first example of calculation, the evaluated amount is calculated based on the correlation between a first blood pressure data group including at least one item of blood pressure data corresponding to the single measurement condition and a second blood pressure data group including at least one item of blood pressure data corresponding to another measurement condition, in the blood pressure data stored in memory 12. For example, an in-group average value of the blood pressure data included in the first blood pressure data group and an in-group average value of the blood pressure data included in the second blood pressure data group, are initially calculated. An average value of the calculated in-group average values and a difference value between the calculated in-group average values are then calculated. A difference between the calculated average value and a threshold value predetermined therefor, and a difference between the calculated difference value and a threshold value predetermined therefor, may further be calculated.

More specifically, the evaluated amount, namely, the risk value is calculated as follows. CPU 20A calculates the risk value based on a program for calculating a cardiovascular risk value, which program is stored in advance in an internal memory or memory 12. In order to calculated the risk value, CPU 20A initially reads the blood pressure data stored in memory 12, and performs a process of calculating an average of the blood pressure data for each of regions 26 and 27 shown in Fig. 3A. In other words, an in-group average of a blood pressure data group including blood pressure data measured under the same measurement condition is calculated for each blood pressure data group. The calculated average blood pressure values for the blood pressure data groups are then used to calculate the risk value. An average value of the blood pressure values in region 28 may concurrently be calculated.

Calculation of the average blood pressure value is carried out using the following equations.

$$\text{average of SBP measured after waking up} = (\text{result of SBP1 measured after waking up} + \text{result of SBP2 measured after waking up} + ... + \text{result of SBPn measured after waking up}) / N \ (\text{where } n = 1, 2, 3, ...)$$

$$\text{average of SBP measured before sleeping} = (\text{result of SBP1 measured before}$$
$$\text{sleeping} + \text{result of SBP2 measured before sleeping} + ... + \text{result of SPBm measured}$$
$$\text{before sleeping}) / m \text{ (where } m = 1, 2, 3, ...)$$

In addition, for calculation of the risk value, an average value (ME average value) of the average of the blood pressure values measured in the time slot before sleeping and the average of the blood pressure values measured in the time slot after waking up, and a difference between the two (ME difference), which are calculated in accordance with the following equations, are used.

$$\text{ME difference} = \text{average of SBP measured after waking up} - \text{average of SBP}$$
$$\text{measured before sleeping}$$

$$\text{ME average} = (\text{average of SBP measured after waking up} + \text{average of SBP}$$
$$\text{measured before sleeping}) / 2$$

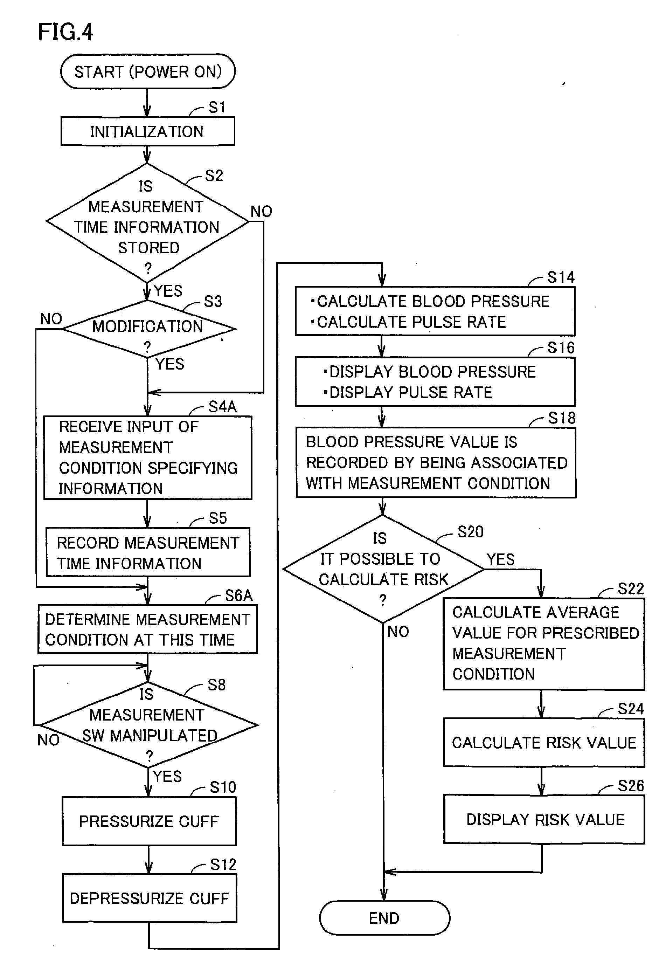

In electronic blood pressure monitor 100 according to the present invention, a process of risk analysis is carried out in accordance with the calculated risk value. Thereby, the blood pressure data group including at least one blood pressure value measured at the time slot before sleeping and the blood pressure data group including at least one blood pressure value measured at the time slot after waking up (data included in before sleeping region 26 and after waking up region 27) are obtained, and averages of the blood pressure values included in the groups are respectively calculated, and then an average value (ME average value) and a difference (ME difference) between the groups, which are two risk values of cardiovascular system diseases, are calculated, so that the risk values are presented (displayed) as a result. In addition to presenting the ME average value and ME difference as such, a difference value between the ME average value and a predetermined threshold value (e.g. 135 mmHg) and a difference value between the ME difference and a predetermined threshold value (e.g. 20 mmHg) may be calculated, and each of the calculated difference values may be presented as a risk value. The risk value may be output by the method as disclosed in Fig. 4 of Japanese Patent Laying-Open No. 2004-261452.

• Second Example of Calculation

In a second example of calculation, an evaluated amount is calculated based on a blood pressure data group corresponding to a prescribed (single) measurement condition. In this case, an in-group average value of blood pressure data included in the blood pressure data group corresponding to the prescribed measurement condition is calculated as an evaluated amount. Furthermore, an evaluated amount may be calculated based on the calculated in-group average value and any of a predetermined threshold value and a predetermined computational expression.

More specifically, the evaluated amount, namely, the risk value is calculated as follows. As in the case above, CPU 20A calculates a risk value based on a program for calculating a cardiovascular risk value, which program is stored in advance in an internal memory or memory 12. In order to calculate the risk value, CPU 20A initially reads the blood pressure data stored in memory 12, and performs a process of calculating averages of the blood pressure data in region 27 shown in Fig. 3A. In other words, in-group averages of the blood pressure data group including blood pressure data measured under the same measurement condition are calculated for a prescribed measurement condition (the time slot after waking up). The calculated averages of the blood pressure values in the blood data group are then used to calculate the risk value.

$$\text{average of SBP measured after waking up} = (\text{result of SBP1 measured after waking up} + \text{result of SBP2 measured after waking up} + ... + \text{result of SBPn measured after waking up}) / n \text{ (where n = 1, 2, 3, ...)}$$

$$\text{average of DBP measured after waking up} = (\text{result of DBP1 measured after waking up} + \text{result of DBP2 measured after waking up} + ... + \text{result of DPBn measured after waking up}) / n \text{ (where n = 1, 2, 3, ...)}$$

In the second example of calculation, the calculated average of SBP measured after waking up and the calculated average of DBP measured after waking up are calculated as risk values and presented. A difference value between each of the calculated average values and a threshold value predetermined for each of the average values may be calculated, and each of the calculated difference values may be presented as a risk value. As to the threshold value herein, the threshold value for the average of SBP measured after waking up may be set to 135 mmHg, and the threshold value for the average of DBP measured after waking up may be set to 85 mmHg, based on, for example, a criterion defined by the Joint National Committee on Prevention, Detection, Evaluation, and Treatment of High Blood Pressure, or a hypertension criterion of home blood pressure defined by the Japanese Society of Hypertension.

As such, blood pressure is affected by various factors and varies, and hence by calculating an average value for each measurement condition or for a prescribed measurement condition, accuracy of risk estimation is improved.

In the following description, blood pressure monitor 100 in the first embodiment of the present invention calculates a risk value according to the above-described first example of calculation.

(Operation of Electronic Blood Pressure Monitor in the First Embodiment)

Fig. 4 is a flowchart of a main routine executed by CPU 20A in electronic blood pressure monitor 100 in the first embodiment of the present invention. The flowchart in Fig. 4 is stored in advance as a program in memory 12, and read by CPU 20A for execution. Processes shown in Fig. 4 are the ones initiated when, for example, power switch 5 is manipulated so that CPU 20A is supplied with electric power via power unit 25.

Referring to Fig. 4, as an initialization process for electronic blood pressure monitor 100, CPU 20A initially controls each unit, exhausts air from bladder 21, and corrects pressure sensor 14 to 0 mmHg (step S (hereinafter abbreviated as "S") 1). Next, CPU 20A determines whether or not measurement time information has already been stored in, for example, time correlation table 121 which will be described below, in memory 12 (S2). If the measurement time information has already been stored (YES in S2), it is then determined whether or not there is an instruction for modification from a patient (S3). If the instruction for modification is sensed (YES in S3), the process proceeds to S4A. If the instruction for modification is not sensed (NO in S3), the process proceeds to S6A.

In contrast, if the measurement time information has not yet been stored in S2 (NO in S2), the process proceeds to S4A.

In S3, measurement time information in a current state may be displayed on display unit 4 so as to allow the patient to determine whether or not a modification is required. In addition, a default value may be stored in time correlation table 121 so that the determination process in S2 is eliminated. By doing so, an evaluated amount having high reliability is calculated for the patient who has an ordinary life pattern, even if measurement time information is not input.

In S4A, CPU 20A uses time receiving unit 203A to receive an input of determination criterion information included in measurement condition specifying information, namely, an input of measurement time information. Time receiving unit 203A records the received measurement time information in time correlation table 121 in memory 12, and updates the contents of the table (S5).

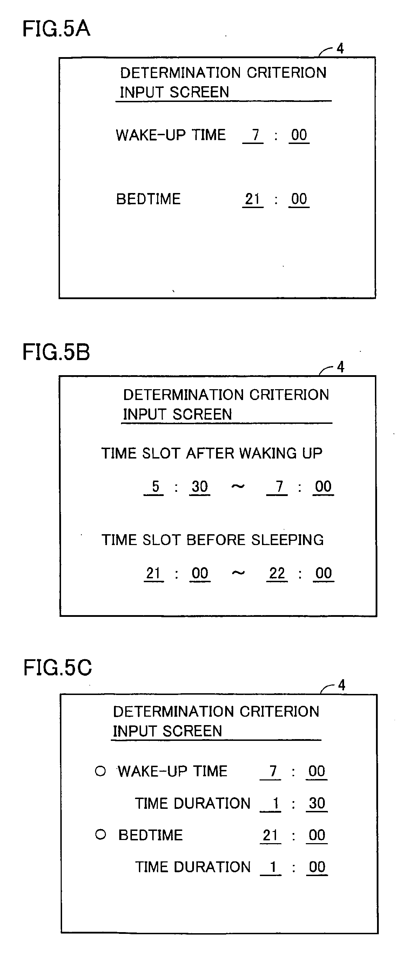

In S4A, a screen for inputting the measurement time information, for example, is displayed on display unit 4. Concrete examples of the screen to be displayed are shown in Figs. 5A, 5B and 5C.

Fig. 5A is a diagram showing an example in which an input of commencement or termination of a time period (measurement time slot) of at least one measurement condition is received as the measurement time information. For example, a "determination criterion input screen" is displayed on display unit 4, so that display for allowing the patient to input wake-up time (e.g. 7:00) and bedtime (e.g. 21:00) is provided. Here, wake-up time is commencement of the measurement condition: time slot after waking up, while bedtime is termination of the measurement condition: time slot before sleeping. Note that any of wake-up time and bedtime may be commencement or termination. In this case, a time duration is determined in advance for each of wake-up time and bedtime, or common time duration is determined for wake-up time and bedtime. For example, an input of commencement or termination of the measurement condition: normal time slot may be received.

Fig. 5B is a diagram showing an example in which an input of commencement and termination of a time period of at least one measurement condition is received as the measurement time information. The "determination criterion input screen" is similarly displayed on display unit 4, and display for allowing the patient to input commencement (e.g. 5:30) and termination (e.g. 7:00) of the time slot after waking up, and commencement (e.g. 21:00) and termination (e.g. 22:00) of the time slot before sleeping is provided.

Fig. 5C is a diagram showing an example in which an input of commencement or termination and time duration (time period duration) of a time period of at least one measurement condition is received as the measurement time information. The "determination criterion input screen" is similarly displayed on display unit 4, and display for allowing the patient to input wake-up time (e.g. 7:00) and bedtime (e.g. 21:00), and time durations thereof (e.g. 1:30, 1:00) is provided. In this case, wake-up time is also commencement of the measurement condition: time slot after waking up, while bedtime is termination of the measurement condition: time slot before sleeping. Note that any of wake-up time and bedtime may be commencement or termination.

In the present embodiment, one switch or more than two switches included in manipulation unit 210 is/are used for the input of each of the times. For example, whenever memory switch 7 is pressed, time may be incremented or decremented by one minute, and the time displayed when power switch 5 is pressed may be determined as input time. A switch for setting time point, not shown, may be provided to input each of the times. Furthermore, a screen displayed when the measurement time information is input is not limited to the forms shown in Figs. 5A-5C.

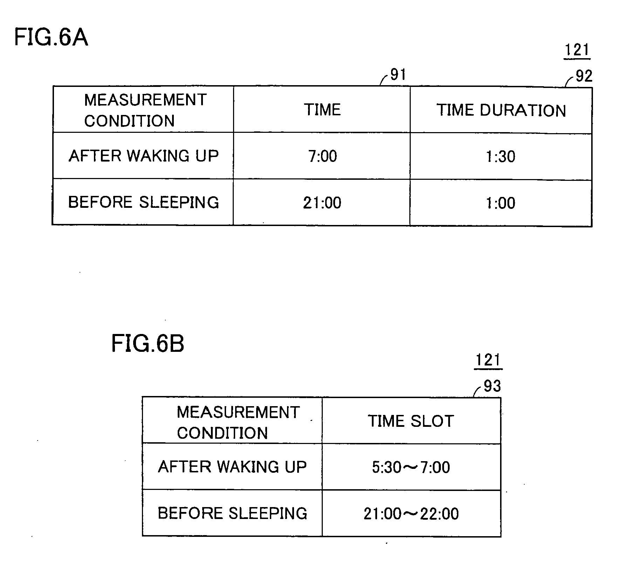

Next, each of Figs. 6A and 6B shows an example of the contents of time correlation table 121 where the measurement time information is stored by the process in S5. Fig. 6A is a first example of the contents of time correlation table 121. In the first example, each of the measurement condition: time slot after waking up and the measurement condition: time slot before sleeping is associated with time data 91 and time duration data 92 and stored in time correlation table 121 in memory 12. Time duration data 92 may be time duration data input by the patient, or data on predetermined time duration. Similar data may be calculated and stored for the measurement condition: normal time slot.

Fig. 6B is a second example of the contents of time correlation table 121. In the second example, each of the measurement condition: time slot after waking up and the measurement condition: time slot before sleeping is paired with time slot data 93 and stored in time correlation table 121 in memory 12. For the measurement condition: normal time slot, data on the time slot other than the time slot after waking up and the time slot before sleeping may similarly be stored.

In the present embodiment, the measurement condition is associated with the measurement time information in time correlation table 121. However, the storage scheme is not limited to such a table form.

If an evaluated amount (risk value) is calculated by the above-described second example of calculation, what is only required is that the time slot after waking up can be distinguished from other time slots. Accordingly, for example, only the input of commencement of the time slot after waking up (wake-up time) or only the input of commencement (wake-up time) and time duration of the time slot after waking up may be received, and stored and updated in memory 12.

Referring to Fig. 4 again, CPU 20A uses determining unit 202A in S6A to obtain timing data output from timer 13, so as to determine the measurement condition at this time. In other words, CPU 20A retrieves time correlation table 121 and determines which measurement condition, namely, which measurement time slot section the current time point belongs to.

Next, CPU 20A determines whether or not measurement switch (reffered to as "SW" in Fig. 4) 6 is manipulated (S8). Determination in S8 is repeated until measurement switch 6 is manipulated. If it is sensed that measurement switch 6 is manipulated, the process proceeds to S 10. The processes in S2-S6A and the process in S8 may be performed in an inverse order.

In S10, CPU 20A controls each unit and applies pressure of up to approximately the systolic blood pressure of the patient + 40 mmHg. Pressure in bladder 21 is then gradually reduced (S12). In this depressurizing process, pressure in bladder 21 is detected by pressure sensor 14, and based on the detected pressure, CPU 20A calculates blood pressure (systolic blood pressure and diastolic blood pressure) values and a pulse rate (S 14). The calculated blood pressure values and pulse rate are displayed on display unit 4 (S16). The processes in S10-S14 for blood pressure measurement are similar to those in the conventional electronic blood pressure monitor. Although blood pressure is measured during a depressurizing process, it may be measured during a pressurizing process.

When calculation and display of blood pressure is terminated, CPU 20A registers new record R, where a measurement result (blood pressure values and a pulse rate) is stored, in a region of memory 12 corresponding to the measurement condition determined in S6A (S 18).

Subsequently, CPU 20A determines whether or not sufficient number of items of data, namely, at least one item of data, for example, is/are recorded in specific regions in memory 12, namely, regions 26 and 27, to allow risk value calculation (S20). If CPU 20A determines that the sufficient number of items of data are not recorded (NO in S20), it terminates a series of processes. In contrast, if CPU 20A determines that the sufficient number of items of data are recorded in specific regions to allow risk value calculation (YES in S20), it calculates an average value for a prescribed measurement condition, namely, an average value of a data group in each of regions 26 and 27 in accordance with the above-described procedure (S22). Then CPU 20A calculates an ME average value and an ME difference as the risk values of cardiovascular system diseases (S24), and displays the calculated risk values at display unit 4 (S26). As such, a series of processes is terminated.

As described above, in the present embodiment, a measurement condition at blood pressure measurement is determined based on an attribute at blood pressure measurement, namely, time point data from timer 13, and measurement time information recorded in advance or input in time correlation table 121 in memory 12. Instead of the attribute at blood pressure measurement, the determined measurement condition is then associated with a measurement result and stored. By doing so, it is possible to calculate an evaluated amount having high accuracy in accordance with the patient's living cycle.

When the patient once sets measurement time information, he/she is not required to input the measurement time information for the second time or more. It is therefore possible to remove the burden of manipulation from the patient whose living cycle is constant. When measurement is carried out for the second time or more, or there is no need of modification, time required for a series of processes in relation to blood pressure measurement can be reduced.

In the present embodiment, when memory switch 7 is manipulated between the prescribed steps, an interrupt process is performed. In the interrupt process, measurement results stored in memory 12, for example, are sequentially read and displayed on display unit 4. In the interrupt process, the above-described processes in S20-S26 may also be performed to display the risk value.

In the present embodiment, the processes in S20-S26 are performed whenever blood pressure is measured. However, the main routine may be terminated in the process in S 18, and the processes in S20-S26 may be performed only in the interrupt process. In addition, the processes in S20-S26 may be performed only when, for example, the patient presses a prescribed switch.

An alarming function may be added to electronic blood pressure monitor 100 according to the present embodiment so that buzzer 24 can generate sound at the set wake-up time. In other words, when the time point obtained from timer 13 reaches the wake-up time set by the patient, CPU 20A may transmit a control signal to buzzer 24 so that an alarm is generated.

[First Modification of First Embodiment]

A first modification of the first embodiment of the present invention will now be described. A configuration of an electronic blood pressure monitor according to the first modification of the first embodiment is similar to that of the first embodiment, and hence the electronic blood pressure monitor denoted by a character 100 shown in Figs. 1 and 2 is also used here for the description.

In the first embodiment, when the determination criterion information (measurement time information) included in the measurement condition specifying information is once input, an input of the relevant information is not received until the instruction for modification is received. In the first modification, however, an input of determination criterion information is received from the patient whenever measurement is performed. In the first modification, measurement time information is similarly input as the determination criterion information.

In the following, an operation of electronic blood pressure monitor 100 in the first modification will be described.

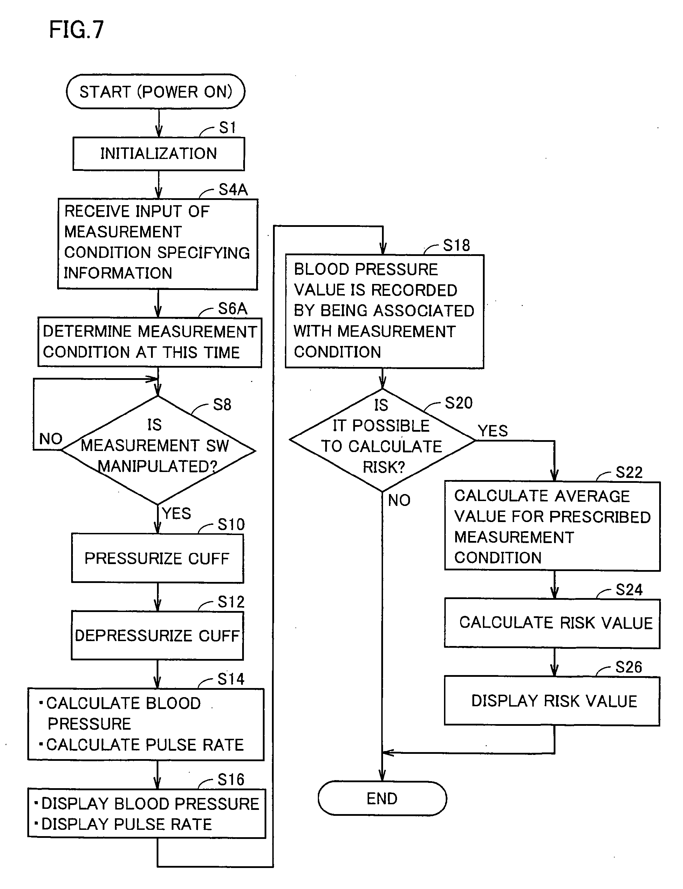

Fig. 7 is a flowchart of a main routine executed by CPU 20A in electronic blood pressure monitor 100 in the first modification of the first embodiment of the present invention. The flowchart in Fig. 7 is stored in advance as a program in memory 12, and read by CPU 20A for execution. Processes similar to those shown in the flowchart in Fig. 4 are denoted by the same characters, and the description thereof will not be repeated here.

Referring to Fig. 7, when the initialization process in S 1 is terminated, CPU 20A receives an input of determination criterion information, namely, measurement time information (S4A). In the first modification, CPU 20A temporarily records the received measurement time information in an internal memory in S4A. When the process in S4A is terminated, the above-described processes in S6A-S26 are sequentially performed so that a series of processes is terminated. In the first modification, the measurement condition at this time is determined in S6A, based on time point data output from timer 13 and measurement time information input in S4A.

As such, electronic blood pressure monitor 100 in the first modification of the first embodiment receives an input of determination criterion information whenever blood pressure is measured. It is therefore possible to present an evaluated amount (risk value) having high reliability even to the patient who has an irregular living cycle.

[Second Modification of First Embodiment]

A second modification of the first embodiment of the present invention will now be described. A configuration of an electronic blood pressure monitor according to the second modification of the first embodiment is similar to that of the first embodiment, and hence the electronic blood pressure monitor denoted by a character 100 shown in Figs. 1 and 2 is also used here for the description.

In the first embodiment and its first modification, an input of determination criterion information is received before blood pressure measurement. In the second modification, however, the input of determination criterion information is received after blood pressure measurement. The second modification is described on the understanding that an input of determination criterion condition is received whenever blood pressure is measured, as in the first modification. However, as in the first embodiment, the input of determination criterion information may be received only when blood pressure is measured for the first time, and when an instruction for modification to the measurement condition is received from the patient. In the second modification, measurement time information is similarly input as determination criterion information.

In the following, an operation of electronic blood pressure monitor 100 in the second modification will be described.

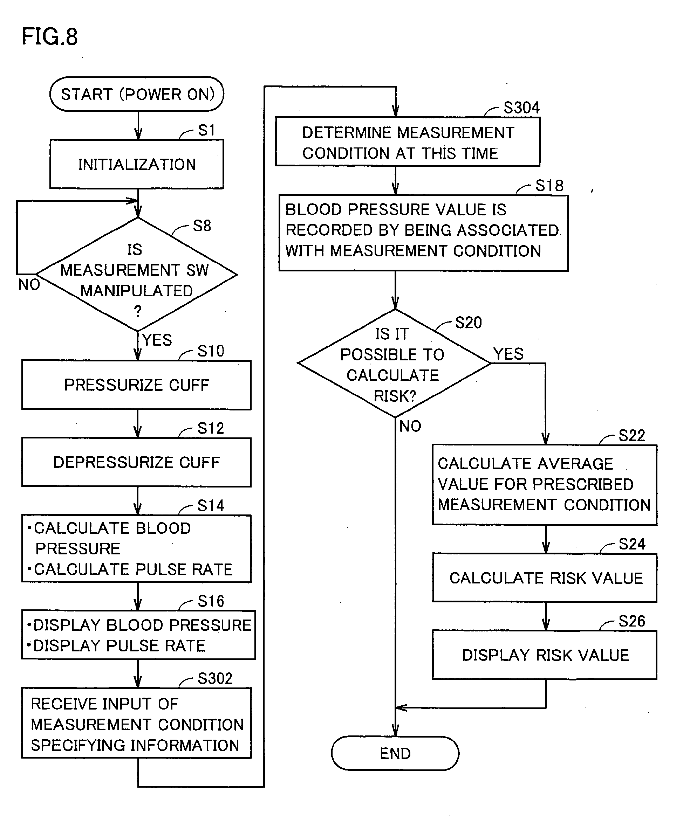

Fig. 8 is a flowchart of a main routine executed by CPU 20A in electronic blood pressure monitor 100 in the second modification of the first embodiment of the present invention. The flowchart in Fig. 8 is stored in advance as a program in memory 12, and read by CPU 20A for execution. Processes similar to those shown in the flowchart in Fig. 4 are denoted by the same characters, and the description thereof will not be repeated here.

Referring to Fig. 8, when the initialization process in S 1 is terminated, the process proceeds to S8 so that it is determined whether or not measurement switch 6 is manipulated. Subsequently, the processes in S10-S16 relating to blood pressure measurement are performed as in the first embodiment.

In S16, after blood pressure values and a pulse rate are displayed on display unit 4, CPU 20A receives an input of determination criterion information (S302). In the second modification, CPU 20A similarly records the received measurement time information temporarily in the internal memory in S4A. The measurement condition at this time is then determined (S304). The processes in S302 and S304 correspond to the processes in S4A and S6A in Fig. 4, respectively, and hence the description thereof will not be repeated.

When the process in S304 is terminated, the processes in S18-S26 are performed as in the first embodiment, and a series of processes is terminated.

[Third Modification of First Embodiment]

A third modification of the first embodiment of the present invention will now be described. A configuration of an electronic blood pressure monitor according to the third modification of the first embodiment is similar to that of the first embodiment, and hence the electronic blood pressure monitor denoted by a character 100 shown in Figs. 1 and 2 is also used for the description.

In the first embodiment and its first and second modifications, the measurement result and the measurement condition are automatically associated with each other and recorded in memory 12 whenever blood pressure is measured. In the third modification, however, these are associated with each other and recorded in memory 12 only when the patient inputs determination criterion information. In the third modification, measurement time information is similarly input as determination criterion information.

In the following, an operation of electronic blood pressure monitor 100 in the third modification will be described.

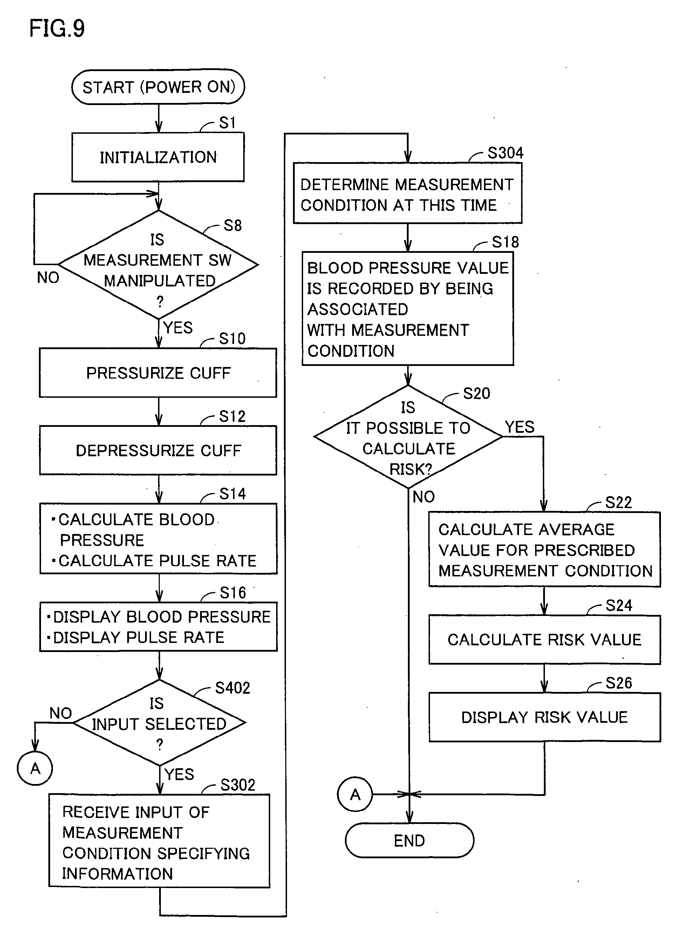

Fig. 9 is a flowchart of a main routine executed by CPU 20A in electronic blood pressure monitor 100 in the third modification of the first embodiment of the present invention. The flowchart in Fig. 9 is stored in advance as a program in memory 12, and read by CPU 20A for execution. Processes similar to those shown in the flowchart in Fig. 8 used for the second modification of the first embodiment are denoted by the same characters, and the description thereof will not be repeated here.

Referring to Fig. 9, when the initialization process in S 1 is terminated, the process proceeds to S8 as in the second modification. When it is sensed that measurement switch 6 is manipulated in S8 (YES in S8), the processes in S10-S16 relating to blood pressure measurement are performed.

After blood pressure values and a pulse rate are displayed on display unit 4 in S16, CPU 20A determines whether or not inputting of determination criterion information is selected (S402). At that time, information that allows the patient to select whether or not he/she inputs the determination criterion information may be displayed on display unit 4. For example, a message "Do you intend to input determination criterion information?" and buttons "YES" and "NO" may be displayed. In this case, CPU 20A senses which button is selected, based on, for example, a manipulate signal from manipulation unit 210. Alternatively, it may be predetermined that, when a prescribed switch such as measurement switch 6 is once manipulated, it is determined that inputting of the condition is selected.

In S402, when CPU 20A determines that inputting of determination criterion information is selected (YES in S402), it receives an input of the determination criterion information (S302). Successively, the measurement condition determining process (S304) is performed, so that the measurement result and the determined measurement condition are associated and stored in memory 12 (S18). When the process in S 18 is terminated, the above-described processes in S20-S26 are performed, and a series of processes is terminated.

In contrast, if CPU 20A determines that inputting of the condition is not selected in S402 (NO in S402), a series of processes is terminated. In other words, if the patient does not select to input the condition, the processes after S302, namely, the measurement condition determining process (S304), the data storing process (S18), and the risk calculating process (S24), for example, are not performed.

In the flowchart in Fig. 9, if inputting of the determination criterion information is not selected (NO in S402), all the processes after S302 are not performed and a series of processes is terminated. However, the processes in S20-S26 relating to risk value calculation may be performed based on the previous measurement value.

As in the first embodiment and its first modification, an input of determination criterion information may be received before measurement. For example, the processes in S402, S302, and S304 may be performed between S1 and S8, so that the processes after S 18 may be performed if YES in S402, or a series of processes may be terminated if NO in S402.

As such, in the third modification, the measurement result and the measurement condition are associated and stored in memory 12 only when the patient selects to input determination criterion information. Accordingly, in the case where the reliability of the measurement result is low owing to extremely insufficient sleep even if the measurement condition corresponds to the time slot after waking up, for example, the measurement result and the measurement condition can be prevented from being associated and stored in memory 12. By doing so, the measurement condition can exactly be associated with the measurement result, and reliability of the risk value to be calculated can be improved.

[Fourth Modification of First Embodiment]

A fourth modification of the first embodiment of the present invention will now be described. A configuration of an electronic blood pressure monitor according to the fourth modification of the first embodiment is similar to that of the first embodiment, and hence the electronic blood pressure monitor denoted by a character 100 shown in Figs. 1 and 2 is also used here for the description.

In the first embodiment and its first to third modifications, blood pressure measurement and inputting of determination criterion information included in measurement condition specifying information are performed in a series of processes (main routine). In the fourth modification, however, they are performed separately. In the fourth modification, measurement time information is similarly input as determination criterion information.

In the following, an operation of electronic blood pressure monitor 100 in the fourth modification will be described.

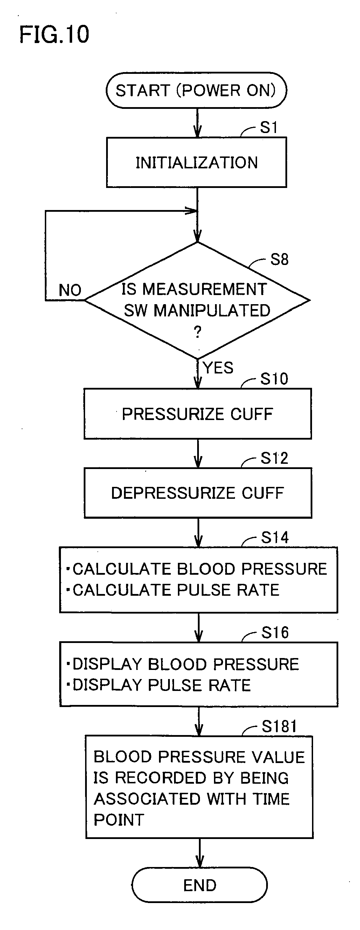

Fig. 10 is a flowchart of a main routine executed by CPU 20A in electronic blood pressure monitor 100 in the fourth modification of the first embodiment of the present invention. The flowchart in Fig. 10 is stored in advance as a program in memory 12, and read by CPU 20A for execution. Processes similar to those shown in the flowchart in Fig. 8 used for the second modification of the first embodiment are denoted by the same characters, and the description thereof will not be repeated here.

Referring to Fig. 10, as in the second modification, the process proceeds to S8 when the initialization process in S 1 is terminated. When it is sensed that measurement switch 6 is manipulated in S8 (YES in S8), the processes in S10-S16 relating to blood pressure measurement are performed.

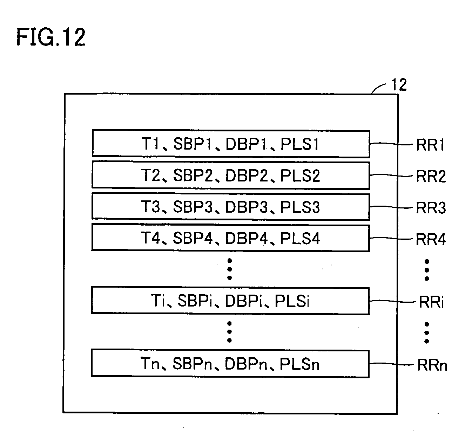

After blood pressure values and a pulse rate are displayed on display unit 4 in S16, CPU 20A obtains time point data from timer 13, and associates the blood pressure values and the pulse rate calculated in S 14 with the time point and records the same in memory 12 (S181). As such, in the fourth modification, the measurement result is associated with the actual time point and stored. The example of how the measurement result in this case is stored is shown in Fig. 12.

Referring to Fig. 12, a record RRi (i = 1, 2, 3, ... , n) is stored at each blood pressure measurement. In record RRi, measurement time point data Ti, systolic blood pressure data SBPi, diastolic blood pressure data DBPi, and pulse rate data PLSi are stored. As such, in the fourth modification, the measurement time point itself, which is an attribute at blood pressure measurement, is associated with the measurement result and stored.

In the fourth modification, when the storing process in S 181 is terminated, a series of processes is terminated.

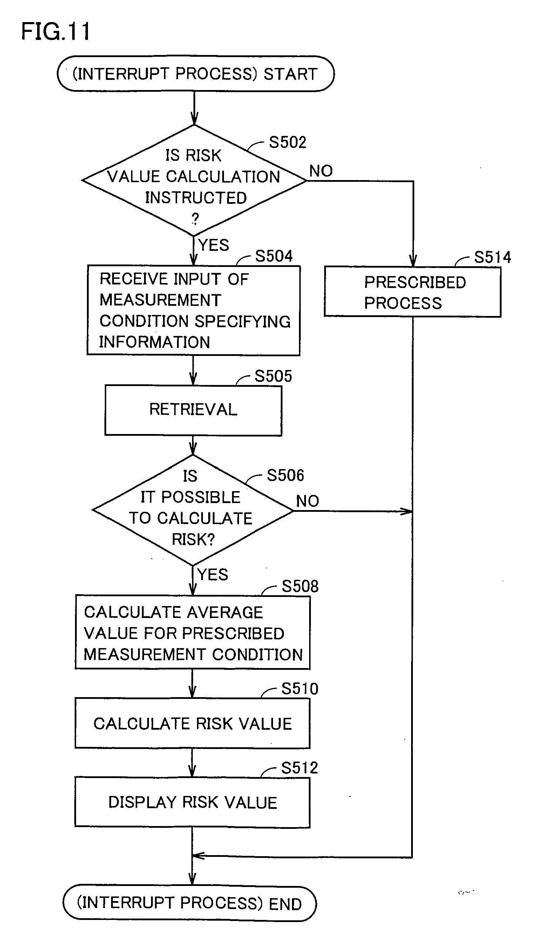

Fig. 11 is a flowchart showing an interrupt process in the fourth modification of the first embodiment. The flowchart in Fig. 11 is also stored in advance as a program in memory 12, and read by CPU 20A for execution. The interrupt process is initiated by memory switch 7 being manipulated.

Referring to Fig. 11, CPU 20A initially determines whether or not there is an instruction for risk value calculation, based on, for example, a manipulate signal from manipulation unit 210 (S502). If there is no instruction for risk value calculation (NO in S502), a prescribed process (S514) such as a process of sequentially reading the measurement results based on measurement time point data Ti and displaying the same is performed, and the interrupt process is terminated.

In contrast, if it is determined that there is an instruction for risk value calculation in S502 (YES in S502), CPU 20A receives an input of determination criterion information, namely, measurement time information (S504). The process in S504 corresponds to the above-described process in S4A, and hence the description thereof will not be repeated here. When the process in S504 is terminated, records RRi corresponding to the measurement conditions: time slot after waking up and time slot before sleeping are retrieved, based on the input time information and time point data Ti (S505).

Next, it is determined whether or not the risk can be calculated (S506). In S506, CPU 20A determines whether or not at least one record RRi exists for each of the prescribed measurement conditions, namely, the time slot after waking up and the time slot before sleeping. If it is determined that the risk can be calculated (YES in S506), an average value for each of the prescribed measurement conditions is calculated (S508), and a risk value is calculated (S510). The calculated risk value is then displayed on display unit 4 (S512). The processes in S508, S510, and S512 are similar to those in S22, S24, and S26 described in the first embodiment, respectively, and hence the description thereof will not be repeated. In contrast, if it is determined that the risk cannot be calculated (NO in S506), the interrupt process is terminated.

In the fourth modification, the risk value is calculated and displayed when there is an instruction for risk value calculation in S502 after the interrupt process is initiated. However, the process in S502 may be eliminated and the processes in S504-S512 may be performed.

As described above, when the evaluated amount (risk value) is calculated in the fourth modification of the first embodiment, an attribute at blood pressure measurement, which attribute is based on an actual measurement time point, is replaced with the measurement condition. By doing so, it is possible to calculate a risk value having high reliability, as in the first embodiment and its first to third modifications.

[Fifth Modification of First Embodiment]

A fifth modification of the first embodiment of the present invention will now be described. A configuration of an electronic blood pressure monitor according to the fifth modification of the first embodiment is similar to that of the first embodiment, and hence the electronic blood pressure monitor denoted by a character 100 shown in Figs. 1 and 2 is also used here for the description.

In the first embodiment, an input of measurement time information is received as determination criterion information included in measurement condition specifying information. In the fifth modification, however, an input of at least one item of manipulation information from information on a manipulation sequence of manipulation unit 210, information on the number of manipulations of manipulation unit 210, and information on a manipulation interval of manipulation unit 210, is received as determination criterion information. In other words, an input of information on a manipulation sequence of a plurality of switches included in manipulation unit 210 and/or information on the number of manipulations of at least one switch and/or information on a manipulation interval of at least one switch is received.

In the following, an operation of electronic blood pressure monitor 100 in the first modification will be described.

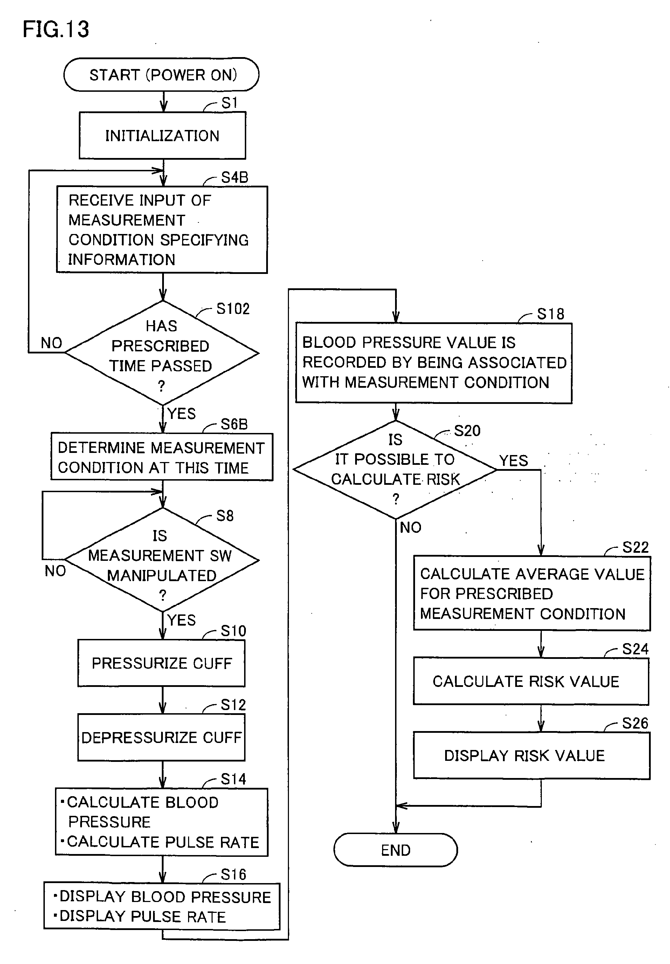

Fig. 13 is a flowchart of a main routine executed by CPU 20A in electronic blood pressure monitor 100 in the fifth modification of the first embodiment of the present invention. The flowchart in Fig. 13 is stored in advance as a program in memory 12, and read by CPU 20A for execution. Processes similar to those shown in the flowchart in Fig. 7 used for the first modification of the first embodiment are denoted by the same characters, and the description thereof will not be repeated here.

Referring to Fig. 13, when the initialization process in S1 is terminated, CPU 20A receives an input of determination criterion information (S4B), and determines whether or not a predetermined time (e.g. 5 seconds) has passed (S102). CPU 20A receives the input of determination criterion information until the predetermined time has passed (NO in S102). When it is determined that the prescribed time has passed (YES in S 102), determining unit 202A is used to determine the measurement condition at this time (S6B). When the process in S6B is terminated, the processes in S8-S26 are performed as in the first embodiment and its first modification.

(Manipulation Sequence)

Initially, an example in which an input of information on a manipulation sequence of a plurality of switches is received as determination criterion information in S4B, will be described. In this example, a correlation table 122 of information on a manipulation sequence and measurement condition information is stored in advance in, for example, memory 12. For example, if the manipulation sequence refers to the order of power switch 5, memory switch 7, and measurement switch 6, the measurement condition is associated with the condition after waking up, and the like. Note that the storage scheme is not limited to correlation table 122 with which information on a manipulation sequence and measurement condition information are associated, as long as these pieces of information are associated.

In this example, CPU 20A senses in S4B which switch among the plurality of switches is manipulated, based on a signal from manipulation unit 210. For example, control switch identifying information and the like is temporarily recorded in the internal memory according to a sensed order.

When the prescribed time has passed, CPU 20A uses determining unit 202A and refers to correlation table 122 as described above, so as to determine the measurement condition in S6B. In other words, correlation table 122 is retrieved based on the identifying information in the sensed order, which identifying information is recorded in the internal memory in S4B, so that information on a corresponding measurement condition is read (determined).

(Number of Manipulations)

Next, an example in which an input of information on the number of manipulations of one switch is received as determination criterion information in S4B, will be described. In this example, a correlation table 123 of information on the number of manipulations and measurement condition information is stored in advance in, for example, memory 12. For example, the measurement condition is associated with the condition after waking up if the number of manipulations of the measurement switch 6 is three. The measurement condition is associated with the condition before sleeping if the number is two. The measurement condition is associated with the normal condition if the number is zero. The storage scheme is not limited to correlation table 123 with which information on the number of manipulations and measurement condition information are associated, as long as these pieces of information are associated.

In this example, CPU 20A sensed in S4B whether or not measurement switch 6, for example, is manipulated based on the manipulate signal from manipulation unit 210. CPU 20A temporarily counts the number of sensed manipulate signals in the internal memory.

When the prescribed time has passed, CPU 20A uses determining unit 202A and refers to correlation table 123 as described above, so as to determine the measurement condition in S6B. In other words, correlation table 123 is retrieved based on the number of manipulations recorded in the internal memory in S4B, so that a corresponding measurement condition is read (determined).

(Manipulation Sequence and Number of Manipulations)

Next, an example in which an input of information on a manipulation sequence of the plurality of switches and information on the number of manipulations of the switches is received as determination criterion information in S4B, will be described. In this example, a correlation table 124 of information on a manipulation sequence and information on the number of manipulations, and measurement condition information, is stored in advance in, for example, memory 12. For example, the measurement condition is associated with the condition after waking up if power switch 5 is manipulated twice and memory switch 7 is subsequently manipulated once. The measurement condition is associated with the condition before sleeping if power switch 5 is manipulated once and memory switch 7 is subsequently manipulated twice. The measurement condition is associated with the normal condition if power switch 5 is manipulated once and memory switch 7 is subsequently manipulated once. The storage scheme is not limited to correlation table 124 with which information on a manipulation sequence and measurement condition information are associated, as long as these pieces of information are associated.

In this example, CPU 20A senses in S4B which switch among the plurality of switches is manipulated, based on a signal from manipulation unit 210. Control switch identifying information and the like is temporarily recorded in the internal memory in, for example, the sensed order.

When the prescribed time has passed, CPU 20A uses determining unit 202A and refers to correlation table 124 as described above, so as to determine the measurement condition in S6B. In other words, a correlation table 124 is retrieved based on the identifying information in a sensed order, which identifying information is stored in the internal memory in S4A, so that a corresponding measurement condition is read (determined).

(Manipulation Interval)

Finally, an example in which an input of information on a manipulation interval, namely, time between manipulations, is received as determination criterion information in S4B, will be described. In this example, a correlation table 125 of information on a manipulation interval and measurement condition information is stored in advance in, for example, memory 12. For example, the measurement condition is associated with the condition after waking up if a time interval at which any one switch or two switches is/are manipulated is 0-2 seconds. The measurement condition is associated with the condition before sleeping if the time interval is 3-5 seconds. The measurement condition is associated with the normal condition if the time interval is 5-10 seconds. The storage scheme is not limited to correlation table 125 with which information on a manipulation interval and measurement condition information are associated, as long as these pieces of information are associated.

In this example, CPU 20A senses in S4B the time between the first and second manipulations, based on the manipulate signal from manipulation unit 210 and the time point data output from timer 13. CPU 20A then temporarily records the sensed time (manipulation interval) in the internal memory.

When the prescribed time has passed, CPU 20A uses determining unit 202A and refers to correlation table 125 as described above, so as to determine the measurement condition in S6B. In other words, correlation table 125 is retrieved based on the time (manipulation interval) recorded in the internal memory in S4B, so that the corresponding measurement condition is read (determined).

Such information on the manipulation interval may be combined with any of the information described above. For example, when a combination of the information on the manipulation interval and information on the number of manipulations is taken as an example, time between manipulations of a prescribed switch is further recorded in S4B. In this case, a correlation table of information on the number of manipulations and the manipulation interval, and the measurement condition, and the like is stored in memory 12. For example, the measurement condition is associated with the condition after waking up if measurement switch 6 is successively manipulated twice, and after a certain time (e.g. 2 second) has passed, measurement switch 6 is again manipulated once.

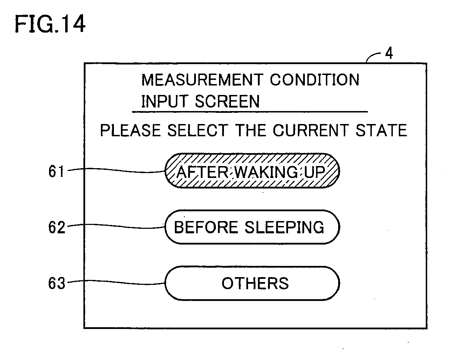

In the fifth modification, an input of at least one item of manipulation information from information on a manipulation sequence of manipulation unit 210, information on the number of manipulations of manipulation unit 210, and information on a manipulation interval of manipulation unit 210, is received as determination criterion information in S4B, and the measurement condition at this time is determined in S6B. However, information on a specific state, namely, a measurement condition itself may be input. An example of display in this case is shown in Fig. 14. In other words, the "specific state" corresponds to the measurement conditions including after waking up, before sleeping, before exercising, after exercising, before meal, after meal, before taking medicine, after taking medicine, and normal.

Fig. 14 is a diagram showing an example in which an input of information on at least one specific state is received as information on a measurement condition itself As in Figs. 5A-5C, a "measurement condition input screen" is displayed on display unit 4, and a button 61 for selecting the measurement condition: time slot after waking up, a button 62 for selecting the measurement condition: time slot before sleeping, and a button 63 for selecting the measurement condition: normal time slot (the measurement condition other than the time slot after waking up and the time slot before sleeping described above) are displayed. One switch or more than two switches included in manipulation unit 210 is/are used, for example, to select these buttons 61-63. As such, it is possible to simplify the process in S6B by inputting the information on a specific state. Two or more states (measurement conditions) such as after waking up and before meal may be selected as measurement condition information.

The fifth modification is described on the understanding that determination criterion information is input before blood pressure measurement. However, as in the second modification, determination criterion information may be input after blood pressure measurement. Alternatively, as in the third modification, the measurement result and the measurement condition may be associated and stored only when determination criterion information is input.

[Second Embodiment]

A second embodiment will now be described. In the first embodiment, the time point data output from timer 13 is used as an attribute for the measurement condition specifying information, while the measurement time information input by the patient is used as the determination criterion information. In the second embodiment, however, voice data externally input, information on a detected amount from a sensor, and the like are used for the measurement condition specifying information.

For example, such voice data and detected amount can be used as an attribute at blood pressure measurement in the second embodiment.

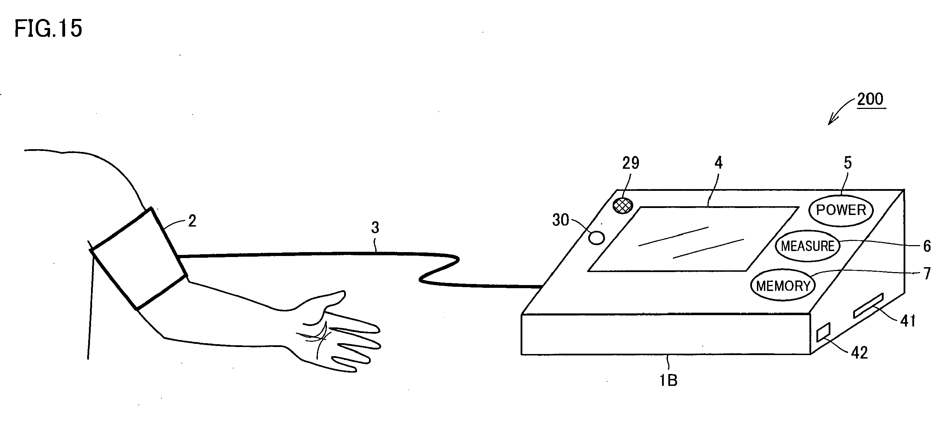

Fig. 15 is a schematic diagram of an electronic blood pressure monitor 200 according to the second embodiment of the present invention. Referring to Fig. 15, electronic blood pressure monitor 200 according to the present embodiment includes a blood pressure monitor main body 1B, cuff 2 attached to a blood pressure measurement site of the patient for pneumatic pressurization, and air pipe 3 connecting blood pressure monitor main body 1B and cuff 2.

Blood pressure monitor main body 1B has an voice receiving unit 29 for receiving an input of voice externally, a sensor 30 for detecting a light quantity, for example, a recording medium attached unit 41 for detachably attaching a recording medium to blood pressure monitor main body 1B externally, and a communication connector unit 42 for detachably attaching a cable (not shown) for providing communication between blood pressure monitor main body 1B and an external device, in addition to display unit 4, power switch 5, measurement switch 6, and memory switch 7.

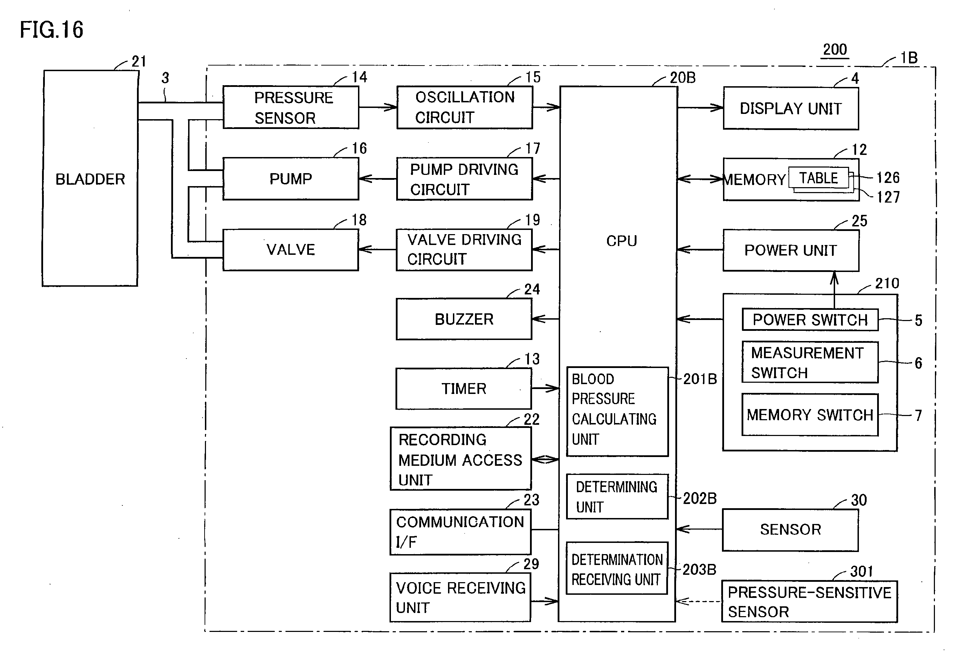

Fig. 16 shows an internal configuration of blood pressure monitor main body 1B. Referring to Fig. 16, blood pressure monitor main body 1B includes a recording medium access unit 22, a communication I/F (abbreviation of interface) 23, voice receiving unit 29, and sensor 30, in addition to the configuration included in blood pressure monitor main body 1A in the first embodiment. A CPU 20B includes a blood pressure calculating unit 201B having a function similar to that of blood pressure calculating unit 201A, and further includes a determining unit 202 B and a detection receiving unit 203B.

As to a recording medium attached to recording medium attached unit 41, recording medium access unit 22 reads or writes data under the control of CPU 20B. Communication I/F 23 communicates with an external device via a cable connected to communication connector unit 42, under the control of CPU 20B.

Voice receiving unit 29 provides the obtained voice data to CPU 20B. CPU 20B uses determining unit 202B to perform a process of recognizing the voice data input from voice receiving unit 29 as an attribute at blood pressure measurement. In the present embodiment, voice is described as a word produced by a person (patient). Accordingly, CPU 20B uses determining unit 202B to, for example, convert the voice data into character data. A correlation table 126 stored in advance as determination criterion information in memory 12, for example, is then retrieved so that the measurement condition is determined. The input voice may be sound or vibration other than a word.

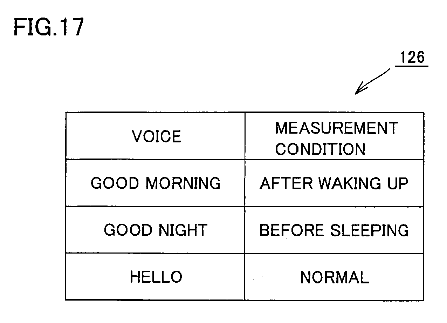

An example of correlation table 126 stored in advance in memory 12 will be described. Fig. 17 shows correlation table 126 of character data and the measurement condition. Referring to Fig. 17, for example, character data "Good Morning" is associated with the measurement condition data "after waking up", while character data "Good Night" is associated with the measurement condition data "before sleeping" in correlation table 126. Furthermore, character data "Hello" is associated with the measurement condition data "normal".

In the second embodiment, such correlation table 126 is stored in advance in electronic blood pressure monitor 200. However, such correlation may be set by the patient or the like. For example, a setting screen may be displayed when blood pressure is measured for the first time, for example, and words for each of the measurement conditions may be registered. Alternatively, if anything other than the word is used, an alarm of an alarm clock, for example, may be associated with the measurement condition: time slot after waking up and registered.

Sensor 30 detects a light quantity of sunlight or the like. Here, a table 127, where each measurement condition and a threshold value of the light quantity are associated and stored, is stored in advance in memory 12. Sensor 30 provides a signal of the detected light quantity to CPU 20B. CPU 20B uses detection receiving unit 203B to receive an input of the detected amount from sensor 30, namely, the light quantity, as an attribute at blood pressure measurement. CPU 20B then uses determining unit 202B to compare the input detected amount and each of the threshold values in table 127 in memory 12, and determines the measurement condition based on the comparison result. For example, if it is determined that the detected amount from sensor 30 exceeds a certain threshold value in table 127, based on the comparison result, the measurement condition corresponding to the certain threshold value is read from table 127, so that the read measurement condition refers to the determined measurement condition. For example, the measurement condition can be determined as the "time slot after waking up".

Alternatively, instead of sensor 30, a pressure-sensitive sensor 301 provided under a mat of the bed, for example, and CPU 20B may be connected so that the detected amount from pressure-sensitive sensor 301 may be input to CPU 20B.

In electronic blood pressure monitor 200 in the second embodiment, a program having a procedure similar to that of the flowchart in Fig. 7 used for the first modification of the first embodiment, for example, is stored in advance in memory 12. CPU 20B reads the program, and thereby performs the processes of determining the measurement condition, measuring blood pressure, calculating the risk value, displaying the risk value, and the like by following a procedure similar to that of the first modification of the first embodiment. In this case, CPU 20B obtains the detected amount from voice receiving unit 29 or sensor 30 (or pressure-sensitive sensor 301) as an attribute in S4A. In S6A, CPU 20B determines the measurement condition based on the voice data or the detected amount, and the predetermined determination criterion information. By doing so, the measurement condition and the measurement result are associated and stored in memory 12 in S 18.

As such, also in the second embodiment, the measurement condition is substituted for the attribute at blood pressure measurement, which attribute is externally input. The substituted measurement condition is associated with the measurement result. It is therefore possible to calculate an evaluated amount having high reliability.

The time point data recorded by timer 13 may be used as the attribute at blood pressure measurement, and an input of the voice data or the detected amount may externally be received as the determination criterion information, as in the first embodiment.

In other words, in the first embodiment, the measurement time information such as commencement or termination of the time slot corresponding to the measurement condition is input by the patient as the determination criterion information. However, the commencement or termination of the time slot may be determined based on the detected amount from sensor 30 or voice receiving unit 29.

For example, when the voice data from voice receiving unit 29 is used as the determination criterion information, a time point at which an alarm of an alarm clock is input may be stored in memory 12 as the commencement of the time slot after waking up. In this case, the time duration of the time slot after waking up is predetermined. By doing so, CPU 20B determines the measurement condition at blood pressure measurement, based on such information stored in advance and the time point data obtained from timer 13.

When the detected amount (light quantity) from sensor 30 is used as the determination criterion information, a threshold value and a time duration for each of the time slot after waking up and the time slot before sleeping are preset in memory 12 in order to specify commencement of the time slot after waking up and commencement of the time slot before sleeping. CPU 20B always obtains the detected amount from sensor 30 and compares the same with these preset threshold values. Based on the comparison result, CPU 20B registers in memory 12 the time point data output from timer 13 as the commencement of the time slot corresponding to the measurement condition. For example, when the detected amount exceeds the threshold value associated with the time slot after waking up, the time point data output from timer 13 is obtained, and the time point data is registered in a prescribed region of memory 12 as the commencement of the time slot after waking up. By doing so, the measurement condition is determined at blood pressure measurement based on such information stored in advance and the time point data obtained from timer 13.

Alternatively, instead of sensor 30, pressure-sensitive sensor 301 provided under a mat of the bed, for example, and CPU 20B may be connected, and commencement and termination of the time slot corresponding to the measurement condition may similarly be determined. In this case, for example, a threshold value and time duration for each of the time slot after waking up and the time slot before sleeping are preset in memory 12 in order to specify the commencement of the time slot after waking up and the termination of the time slot before sleeping. CPU 20B then uses determining unit 202B and always obtains the detected amount (pressure value) from the pressure-sensitive sensor, so as to compare the amount of change in pressure value and these predetermined threshold values. Based on the comparison result, CPU 20B registers in memory 12 the time point data output from timer 13 as the commencement or the termination of the time slot corresponding to the measurement condition. For example, when the amount of change in pressure value exceeds the threshold value associated with the time slot after waking up, the time point data output from timer 13 is obtained, and the time point data is stored in a prescribed region of memory 12 as the commencement of the time slot after waking up. By doing so, the measurement condition is determined as in the case of sensor 30.

[Third Embodiment]

As a third embodiment, a blood pressure measurement system provided with electronic blood pressure monitor 200 shown in the above-described second embodiment and a information-processing device is provided.

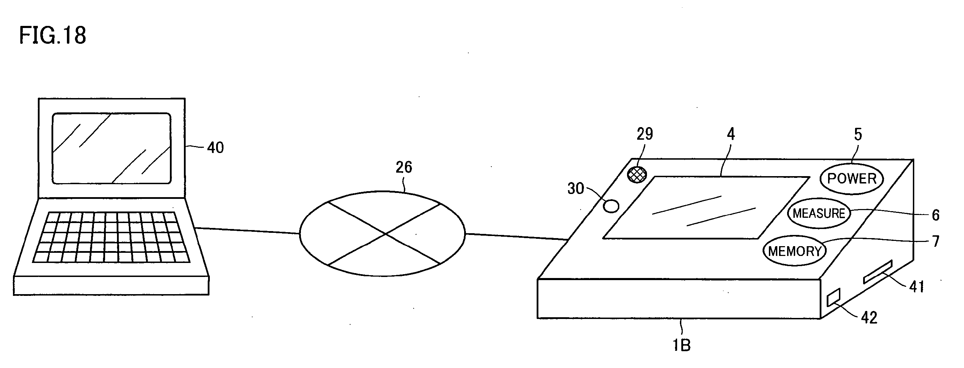

In this system, determination criterion information included in measurement condition specifying information can be input, not by electronic blood pressure monitor 200, but by, for example, a personal computer serving as an external information-processing device. For example, as shown in Fig. 18, an external personal computer 40 is provided with a function of receiving an input of the measurement time information, for example, as the determination criterion information. Electronic blood pressure monitor 200 and personal computer 40 are connected via a communication line 26. Personal computer 40 transfers the input determination criterion information to electronic blood pressure monitor 200 via communication line 26. Electronic blood pressure monitor 200 records in memory 12 the determination criterion information input by communication I/F 23, and performs the processes similar to those of, for example, the first embodiment and its first to fourth modifications. Here, a dedicated line such as a local area network, and a public network such as a telephone line, may be applied to communication line 26.

Furthermore, the function of calculating an average value of each data group and calculating and displaying the risk value may be performed, not by electronic blood pressure monitor 200, but by personal computer 40 or the like. Personal computer 40 is provided with a function of calculating an average value of each data group read from memory 12 and calculating and displaying the risk value. Electronic blood pressure monitor 200 brings itself into a communication enable state by using communication I/F 23 and communication connector unit 42. In this state, when the patient manipulates a communication switch not shown, CPU 20B reads data stored in regions 26-28 of memory 12 and transfers the data to personal computer 40 via communication I/F 23 and communication line 26. Alternatively, only the data required for calculation of the evaluated amount, namely, data in regions 26 and 27, for example, may be read and transferred.

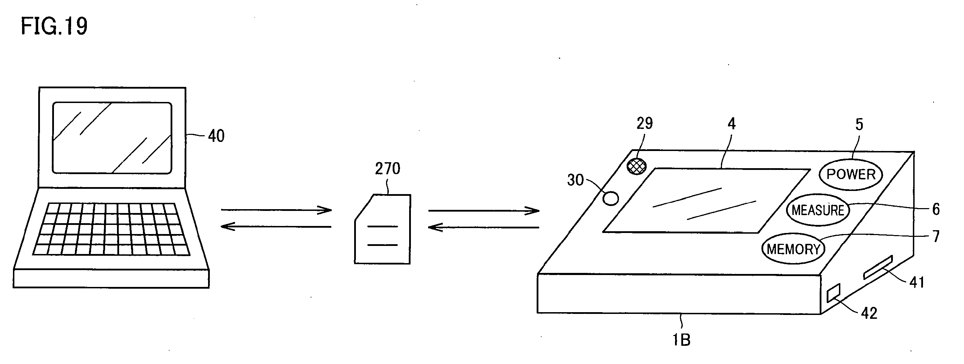

Instead of the data transfer via communication line 26 shown in Fig. 18, a recording medium 270 such as a memory card may be used to receive and transfer determination criterion information and measurement data, as in Fig. 19.

Specifically, the patient inputs determination criterion information to personal computer 40 and records the same in recording medium 270. When recording medium 270 is attached to recording medium attached unit 22 in electronic blood pressure monitor 200, CPU 20B reads the determination criterion information from recording medium 270 and records the same in memory 12. Electronic blood pressure monitor 200 then performs the processes similar to those, for example; of the first embodiment and its first to fourth modifications. Alternatively, when recording medium 270 is attached to recording medium access unit 22, the measurement condition may be determined based on the determination criterion information recorded in recording medium 270 instead of memory 12.

When the measurement data is received and transferred, recording medium 270 is attached to recording medium access unit 22, so that CPU 20B reads data in regions 26-28 of memory 12 and writes the data into recording medium 270 attached to recording medium access unit 22. After the writing, recording medium 270 is removed and attached to personal computer 40, so that the data is read and transferred. When recording medium 270 is attached to recording medium access unit 22, an area where the measurement result is to be stored in S 18 in Fig. 4 may be recording medium 270, instead of memory 12.

In the above-described system, the following operations may also be possible. In electronic blood pressure monitor 200, as shown in Fig. 12 of the fourth modification of the first embodiment, the measurement result and the time point data are associated and stored in record RRi, and all the measurement data (the measurement result and the time point data) is transferred to personal computer 40. Personal computer 40 obtains the measurement data transferred by electronic blood pressure monitor 200. In addition, personal computer 40 receives an input of the measurement time information by the patient or the like, and calculates and displays the risk value as described above.

As to the embodiments of the present invention described above, the description has been made taking as an example an upper arm blood pressure monitor, for which an upper arm is supposed as a measurement site. However, the embodiments can be applied to any blood pressure monitor, as long as it is attached to extremities such as a wrist. In addition, as to the types of the measurement condition in the embodiments of the present invention, the time slot before sleeping, the time slot after waking up, and the normal time slot, which are associated with the rhythm of life, are provided. However, the types and numbers thereof are not limited thereto. For example, the measurement conditions such as before exercising, after exercising, and the like may be provided, and these measurement conditions may be associated with the measurement result. In addition, an evaluated amount may be calculated based on such measurement conditions. Furthermore, a plurality of measurement conditions such as the time slot after waking up and before exercising may be associated with the measurement result.

Although the present invention has been described and illustrated in detail, it is clearly understood that the same is by way of illustration and example only and is not to be taken by way of limitation, the spirit and scope of the present invention being limited only by the terms of the appended claims.

**Claims**

1. An electronic blood pressure monitor, comprising:

    a cuff (2) attachable to a blood pressure measurement site;
    a pressurizing and depressurizing unit (16, 18) for adjusting pressure applied to said cuff;
    a pressure detecting unit (14, 15) for detecting pressure in said cuff;
    a blood pressure calculating unit (201A) for calculating blood pressure from a signal obtained at said pressure detecting unit;
    a storing unit (12) for storing data of the calculated blood pressure; and
    a determining unit (202A) for, based on measurement condition specifying information for specifying a measurement condition at blood pressure measurement, determining at least one corresponding measurement condition among a plurality of types of blood pressure measurement conditions.

2. The electronic blood pressure monitor according to claim 1, further comprising
    a timer unit for recording a time point, and
    a receiving unit for receiving from a subject an input of time information as said measurement condition specifying information, wherein
    said received time information and information on the measurement condition are associated and further stored in said storing unit,
    said determining unit determines said measurement condition based on time point data output from said timer unit and said time information stored in said storing unit, and
    at each blood pressure measurement, said measurement condition determined by said determining unit is related to the data of the blood pressure calculated by said blood pressure calculating unit, and stored in said storing unit.

3. The electronic blood pressure monitor according to claim 2, wherein said determining unit retrieves said information on the measurement condition stored in said storing unit, based on said time point data and said time information, and determines said measurement condition based on a detected result.

4. The electronic blood pressure monitor according to claim 3, wherein
    in said storing unit, a storage region is provided for each of said plurality of types of measurement conditions, and said data of the blood pressure is stored in said storage region corresponding to said information on the measurement condition.

5. The electronic blood pressure monitor according to claim 3, wherein said plurality of types of measurement conditions include a measurement condition indicating any of blood pressure measurement after waking up and blood pressure measurement before sleeping.

**6.** The electronic blood pressure monitor according to claim 2, wherein
said plurality of types of measurement conditions correspond to a "period", the period including time, a day of a week, a day, the week, a month, a season, and a year, and
said time information is information on said period.

**7.** The electronic blood pressure monitor according to claim 2, wherein
said plurality of types of measurement conditions correspond to a "time period", and
said time information is information on commencement of said time period and information on termination of said time period.

**8.** The electronic blood pressure monitor according to claim 2, wherein
said plurality of types of measurement conditions correspond to a "time period", and
said time information is information on any of commencement and termination of said time period, and information on a time period duration.

**9.** The electronic blood pressure monitor according to claim 2, wherein
said plurality of types of measurement conditions correspond to a "time period",
a time period duration of said time period is predetermined, and
said time information is information on any of commencement and termination of said time period.

**10.** The electronic blood pressure monitor according to claim 2, wherein an input of said measurement condition specifying information is received from said receiving unit, either of before blood pressure measurement, after blood pressure measurement, and in a case where inputing after blood pressure measurement is selected.

**11.** The electronic blood pressure monitor according to claim 1, further comprising
a timer unit for recording a time point, and
a receiving unit for receiving from a patient an input of time information as said measurement condition specifying information, wherein
at each blood pressure measurement, the electronic blood pressure monitor relates time point data output from said timer unit to the data of the blood pressure calculated by said blood pressure calculating unit, for storage in said storing unit, and
said determining unit determines said measurement condition based on said received time information and the time point data related to the data of the blood pressure in said storing unit.

**12.** The electronic blood pressure monitor according to claim 1, further comprising a receiving unit for receiving an input of voice information as said measurement condition specifying information, wherein
said determining unit performs a process of recognizing said received voice information, retrieves information on the measurement condition corresponding to said recognized voice information, and determines the measurement condition at said blood pressure measurement based on a retrieval result, and
at each blood pressure measurement, the electronic blood pressure monitor relates said measurement condition determined by said determining unit to the data of the blood pressure calculated by said blood pressure calculating unit, for storage in said storing unit.

**13.** The electronic blood pressure monitor according to claim 1, further comprising a receiving unit for additionally serving as at least one manipulation unit manipulated for receiving an external instruction, and for receiving, as said measurement condition specifying information, an input of at least one piece of manipulation information from information on a manipulation sequence of said manipulation unit, information on a number of manipulations of said manipulation unit, and information on a manipulation interval of said manipulation unit, wherein
said determining unit retrieves information on the measurement condition associated in advance with said received manipulation information, and determines said measurement condition based on a retrieval result, and
at each pressure measurement, said measurement condition determined by said determining unit is related to the data of the blood pressure calculated by said blood pressure calculating unit, and stored in said storing unit.

**14.** The electronic blood pressure monitor according to claim 1, further comprising
a detecting unit for detecting any of a light quantity and a pressure amount, and
a receiving unit for receiving from said detecting unit an input of a detected amount as said measurement condition specifying information, wherein
said determining unit compares said received detected amount and a predetermined threshold value, and determines said measurement condition based on a comparison result, and
at each blood pressure measurement, said measurement condition determined by said determining unit is related to the data of the blood pressure calculated by said blood pressure calculating unit, and stored in said storing unit.

15. A method of determining a measurement condition by using a device including

a cuff (2) attachable to a blood pressure measurement site,
a pressurizing and depressurizing unit (16, 18) for adjusting pressure applied to said cuff,
a pressure detecting unit (14, 15) for detecting pressure in said cuff,
a blood pressure calculating unit (201A) for calculating blood pressure from a signal obtained at said pressure detecting unit, and
a storing unit (12) for storing data of the calculated blood pressure, comprising the steps of:

   receiving measurement condition specifying information for specifying a measurement condition at blood pressure measurement; and
   determining at least one corresponding measurement condition among a plurality of types of said measurement conditions, based on said received measurement condition specifying information.

16. A program for allowing a computer to execute said method of determining the measurement condition recited in claim 15.

17. A machine readable recording medium recording a program for allowing a computer to execute said method of determining the measurement condition recited in claim 15.

EP 1 776 920 A2

FIG.1

100

4   5

POWER

MEASURE   6

MEMORY   7

1A

2

3

FIG.2

100

1A

PRESSURE SENSOR — 14

OSCILLATION CIRCUIT — 15

20A

DISPLAY UNIT — 4

BLADDER — 21

3

PUMP — 16

PUMP DRIVING CIRCUIT — 17

CPU

MEMORY — 12

TABLE — 121
122
123
124
125

VALVE — 18

VALVE DRIVING CIRCUIT — 19

BLOOD PRESSURE CALCULATING UNIT — 201A

POWER UNIT — 25

BUZZER — 24

210

DETERMINING UNIT — 202A

POWER SWITCH — 5

TIMER — 13

MEASUREMENT SWITCH — 6

TIME RECEIVING UNIT — 203A

MEMORY SWITCH — 7

EP 1 776 920 A2

22

## FIG.3A

```
                                    ⌐12
┌──────────────────────────────────────┐
│   ┌──────────────────────────────┐    │
│   │  ┌────────────────────────┐  │──R │
│   │  │   SBP、DBP、PLS          │  │    │
│   │  └────────────────────────┘  │    │
│   │                              │─26 │
│   │   BEFORE SLEEPING            │    │
│   │   MEASUREMENT REGION         │    │
│   └──────────────────────────────┘    │
│   ┌──────────────────────────────┐    │
│   │                              │    │
│   │   AFTER WAKING UP            │─27 │
│   │   MEASUREMENT REGION         │    │
│   │                              │    │
│   └──────────────────────────────┘    │
│   ┌──────────────────────────────┐    │
│   │                              │    │
│   │                              │    │
│   │  NORMAL MEASUREMENT REGION   │─28 │
│   │                              │    │
│   └──────────────────────────────┘    │
└──────────────────────────────────────┘
```

## FIG.3B

```
                                    ⌐12
┌──────────────────────────────────────┐
│   ┌──────────────────────────────┐    │
│   │   SBP1、DBP1、PLS1、C1         │───R1 │
│   ├──────────────────────────────┤    │
│   │   SBP2、DBP2、PLS2、C2         │───R2 │
│   ├──────────────────────────────┤    │
│   │   SBP3、DBP3、PLS3、C3         │───R3 │
│   ├──────────────────────────────┤    │
│   │   SBP4、DBP4、PLS4、C3         │───R4 │
│   └──────────────────────────────┘    │
│                  :                :   │
│   ┌──────────────────────────────┐    │
│   │   SBPi、DBPi、PLSi、C1         │───Ri │
│   └──────────────────────────────┘    │
│                  :                :   │
│   ┌──────────────────────────────┐    │
│   │   SBPn、DBPn、PLSn、C2         │───Rn │
│   └──────────────────────────────┘    │
└──────────────────────────────────────┘
```

EP 1 776 920 A2

# FIG.4

```
        START (POWER ON)
                │
                ▼          S1
        INITIALIZATION
                │
                ▼          S2
             IS
        MEASUREMENT           NO
        TIME INFORMATION ──────────────────────┐
           STORED                               │
             ?                                  │
                │ YES                           │
                ▼          S3                    │
     NO   MODIFICATION                           │
    ┌─────────   ?                               │
    │            │ YES                           │
    │            ▼          S4A                   │
    │    RECEIVE INPUT OF                         │
    │    MEASUREMENT                              │
    │    CONDITION SPECIFYING                     │
    │    INFORMATION                              │
    │            │          S5                    │
    │            ▼                                │
    │    RECORD MEASUREMENT                       │
    │    TIME INFORMATION                         │
    │            │                                │
    └────────────┤          S6A                   │
                 ▼                                │
     DETERMINE MEASUREMENT                        │
     CONDITION AT THIS TIME                       │
                 │                                │
       ┌─────────┤          S8                    │
       │         ▼                                │
       │        IS                                │
       │   MEASUREMENT                            │
       NO   SW MANIPULATED                        │
       │         ?                                │
       │         │ YES                            │
       │         ▼          S10                    │
       │   PRESSURIZE CUFF                         │
       │         │          S12                    │
       │         ▼                                 │
       │   DEPRESSURIZE CUFF                       │
       │         │                                 │
       └─────────┴──────────────────┐             │
                                     ▼             ▼
```

·CALCULATE BLOOD PRESSURE  — S14
·CALCULATE PULSE RATE

·DISPLAY BLOOD PRESSURE  — S16
·DISPLAY PULSE RATE

BLOOD PRESSURE VALUE IS  — S18
RECORDED BY BEING ASSOCIATED
WITH MEASUREMENT CONDITION

IS  — S20
IT POSSIBLE TO    YES
CALCULATE RISK  ──────────┐
?

NO

CALCULATE AVERAGE  — S22
VALUE FOR PRESCRIBED
MEASUREMENT
CONDITION

CALCULATE RISK VALUE  — S24

DISPLAY RISK VALUE  — S26

END

## FIG.5A

DETERMINATION CRITERION
INPUT SCREEN

WAKE-UP TIME   7 : 00

BEDTIME       21 : 00

## FIG.5B

DETERMINATION CRITERION
INPUT SCREEN

TIME SLOT AFTER WAKING UP

5 : 30  ~  7 : 00

TIME SLOT BEFORE SLEEPING

21 : 00  ~  22 : 00

## FIG.5C

DETERMINATION CRITERION
INPUT SCREEN

O  WAKE-UP TIME    7 : 00

      TIME DURATION  1 : 30

O  BEDTIME         21 : 00

      TIME DURATION  1 : 00

## FIG.6A

<u>121</u>

| MEASUREMENT CONDITION | TIME | TIME DURATION |
|---|---|---|
| AFTER WAKING UP | 7:00 | 1:30 |
| BEFORE SLEEPING | 21:00 | 1:00 |

(91) (92)

## FIG.6B

<u>121</u>

| MEASUREMENT CONDITION | TIME SLOT |
|---|---|
| AFTER WAKING UP | 5:30~7:00 |
| BEFORE SLEEPING | 21:00~22:00 |

(93)

## FIG.7

START (POWER ON)

↓ S1

INITIALIZATION

↓ S4A

RECEIVE INPUT OF
MEASUREMENT
CONDITION SPECIFYING
INFORMATION

↓ S6A

DETERMINE MEASUREMENT
CONDITION AT THIS TIME

↓

S8
IS MEASUREMENT SW MANIPULATED?

NO →

YES ↓ S10

PRESSURIZE CUFF

↓ S12

DEPRESSURIZE CUFF

↓ S14

· CALCULATE BLOOD
  PRESSURE
· CALCULATE PULSE RATE

↓ S16

· DISPLAY BLOOD PRESSURE
· DISPLAY PULSE RATE

S18

BLOOD PRESSURE
VALUE IS RECORDED
BY BEING ASSOCIATED
WITH MEASUREMENT
CONDITION

↓

S20
IS IT POSSIBLE TO CALCULATE RISK?

YES →

NO ↓

S22

CALCULATE AVERAGE
VALUE FOR PRESCRIBED
MEASUREMENT
CONDITION

↓ S24

CALCULATE RISK VALUE

↓ S26

DISPLAY RISK VALUE

END

## FIG.8

START (POWER ON)

S1
INITIALIZATION

S8
IS MEASUREMENT SW MANIPULATED ?

NO

YES

S10
PRESSURIZE CUFF

S12
DEPRESSURIZE CUFF

S14
・CALCULATE BLOOD PRESSURE
・CALCULATE PULSE RATE

S16
・DISPLAY BLOOD PRESSURE
・DISPLAY PULSE RATE

S302
RECEIVE INPUT OF MEASUREMENT CONDITION SPECIFYING INFORMATION

S304
DETERMINE MEASUREMENT CONDITION AT THIS TIME

S18
BLOOD PRESSURE VALUE IS RECORDED BY BEING ASSOCIATED WITH MEASUREMENT CONDITION

S20
IS IT POSSIBLE TO CALCULATE RISK?

YES

NO

S22
CALCULATE AVERAGE VALUE FOR PRESCRIBED MEASUREMENT CONDITION

S24
CALCULATE RISK VALUE

S26
DISPLAY RISK VALUE

END

## FIG.9

START (POWER ON)

↓ S1

INITIALIZATION

↓ S8

IS MEASUREMENT SW MANIPULATED ?

NO → (loops back)

YES ↓ S10

PRESSURIZE CUFF

↓ S12

DEPRESSURIZE CUFF

↓ S14

• CALCULATE BLOOD PRESSURE
• CALCULATE PULSE RATE

↓ S16

• DISPLAY BLOOD PRESSURE
• DISPLAY PULSE RATE

↓ S402

IS INPUT SELECTED ?

NO → (A)

YES ↓ S302

RECEIVE INPUT OF MEASUREMENT CONDITION SPECIFYING INFORMATION

→ S304

DETERMINE MEASUREMENT CONDITION AT THIS TIME

↓ S18

BLOOD PRESSURE VALUE IS RECORDED BY BEING ASSOCIATED WITH MEASUREMENT CONDITION

↓ S20

IS IT POSSIBLE TO CALCULATE RISK?

NO ↓    YES →

S22

CALCULATE AVERAGE VALUE FOR PRESCRIBED MEASUREMENT CONDITION

↓ S24

CALCULATE RISK VALUE

↓ S26

DISPLAY RISK VALUE

(A)

END

29

# FIG.10

```
         ┌─────────────────────┐
         │  START (POWER ON)   │
         └──────────┬──────────┘
                    │          ┌─ S1
         ┌──────────▼──────────┐
         │   INITIALIZATION    │
         └──────────┬──────────┘
                    │
    ┌───────────────┤
    │               │                ┌─ S8
    │          ╱────▼─────╲
    │  NO    ╱  IS MEASUREMENT ╲
    └───────╱  SW MANIPULATED    ╲
            ╲        ?          ╱
             ╲─────────────────╱
                    │ YES
                    │          ┌─ S10
         ┌──────────▼──────────┐
         │  ·PRESSURIZE CUFF   │
         └──────────┬──────────┘
                    │          ┌─ S12
         ┌──────────▼──────────┐
         │  DEPRESSURIZE CUFF  │
         └──────────┬──────────┘
                    │          ┌─ S14
         ┌──────────▼──────────────┐
         │ ·CALCULATE BLOOD PRESSURE│
         │ ·CALCULATE PULSE RATE    │
         └──────────┬──────────────┘
                    │          ┌─ S16
         ┌──────────▼──────────────┐
         │ ·DISPLAY BLOOD PRESSURE  │
         │ ·DISPLAY PULSE RATE      │
         └──────────┬──────────────┘
                    │          ┌─ S181
         ┌──────────▼──────────────┐
         │ BLOOD PRESSURE VALUE     │
         │ IS RECORDED BY BEING     │
         │ ASSOCIATED WITH TIME     │
         │ POINT                    │
         └──────────┬──────────────┘
                    │
         ┌──────────▼──────────┐
         │        END          │
         └─────────────────────┘
```

## FIG.11

```
                    ( (INTERRUPT PROCESS) START )
                                 │
                                 ▼
                        ╱────────────────╲        S502
                       ╱   IS RISK         ╲
                      ╱  VALUE CALCULATION   ╲   NO
                      ╲    INSTRUCTED        ╱────────────┐
                       ╲       ?           ╱              │
                        ╲────────────────╱               │
                                 │ YES                    │
                                 ▼        S504            ▼        S514
                     ┌───────────────────────┐   ┌──────────────────┐
                     │ RECEIVE INPUT OF       │   │ PRESCRIBED       │
                     │ MEASUREMENT            │   │ PROCESS          │
                     │ CONDITION SPECIFYING   │   └──────────────────┘
                     │ INFORMATION            │            │
                     └───────────────────────┘            │
                                 │        S505            │
                                 ▼                        │
                     ┌───────────────────────┐           │
                     │      RETRIEVAL         │           │
                     └───────────────────────┘           │
                                 │                        │
                                 ▼                        │
                        ╱────────────────╲    S506        │
                       ╱      IS           ╲              │
                      ╱   IT POSSIBLE        ╲   NO        │
                      ╲   TO CALCULATE       ╱─────────────┼──►
                       ╲     RISK?         ╱               │
                        ╲────────────────╱                │
                                 │ YES                     │
                                 ▼        S508             │
                     ┌───────────────────────┐            │
                     │ CALCULATE AVERAGE      │            │
                     │ VALUE FOR PRESCRIBED   │            │
                     │ MEASUREMENT CONDITION  │            │
                     └───────────────────────┘            │
                                 │        S510             │
                                 ▼                         │
                     ┌───────────────────────┐            │
                     │ CALCULATE RISK VALUE   │            │
                     └───────────────────────┘            │
                                 │        S512             │
                                 ▼                         │
                     ┌───────────────────────┐            │
                     │ DISPLAY RISK VALUE     │            │
                     └───────────────────────┘            │
                                 │◄───────────────────────┘
                                 ▼
                    ( (INTERRUPT PROCESS) END )
```

FIG.12

| 12 |
|---|
| T1、SBP1、DBP1、PLS1 — RR1 |
| T2、SBP2、DBP2、PLS2 — RR2 |
| T3、SBP3、DBP3、PLS3 — RR3 |
| T4、SBP4、DBP4、PLS4 — RR4 |
| ⋮ ⋮ |
| Ti、SBPi、DBPi、PLSi — RRi |
| ⋮ ⋮ |
| Tn、SBPn、DBPn、PLSn — RRn |

# FIG.13

START (POWER ON)

↓ S1

INITIALIZATION

↓ S4B

RECEIVE INPUT OF
MEASUREMENT
CONDITION SPECIFYING
INFORMATION

↓ S102

HAS
PRESCRIBED
TIME PASSED
?

NO ←

YES ↓ S6B

DETERMINE MEASUREMENT
CONDITION AT THIS TIME

↓ S8

IS
MEASUREMENT SW
MANIPULATED
?

NO ←

YES ↓ S10

PRESSURIZE CUFF

↓ S12

DEPRESSURIZE CUFF

↓ S14

·CALCULATE BLOOD
 PRESSURE
·CALCULATE PULSE RATE

↓ S16

·DISPLAY BLOOD PRESSURE
·DISPLAY PULSE RATE

S18

BLOOD PRESSURE VALUE IS
RECORDED BY BEING ASSOCIATED
WITH MEASUREMENT CONDITION

↓ S20

IS
IT POSSIBLE TO
CALCULATE RISK
?

YES →

NO ↓

S22

CALCULATE AVERAGE VALUE
FOR PRESCRIBED
MEASUREMENT CONDITION

↓ S24

CALCULATE RISK VALUE

↓ S26

DISPLAY RISK VALUE

END

33

FIG.14

MEASUREMENT CONDITION
INPUT SCREEN

PLEASE SELECT THE CURRENT STATE

61 —— AFTER WAKING UP

62 —— BEFORE SLEEPING

63 —— OTHERS

FIG.15

EP 1 776 920 A2

FIG.16

# FIG.17

126

| VOICE | MEASUREMENT CONDITION |
|---|---|
| GOOD MORNING | AFTER WAKING UP |
| GOOD NIGHT | BEFORE SLEEPING |
| HELLO | NORMAL |

FIG.18

FIG.19

EP 1 776 920 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004261452 A

**Non-patent literature cited in the description**

- On Morning Hypertension and Risk for Cerebrovascular Accident. **KAZUOMI KARIO.** Journal of Blood . Pressure. sentan igaku-sha Ltd, 01 November 2002, vol. 9, 94-47